# FASCICULE DE BREVET EUROPEEN

(11) **EP 1 507 758 B1**
(45) Date de publication et mention de la délivrance du brevet: **02.11.2005**
(21) Numéro de dépôt: 03752808.0
(22) Date de dépôt: 15.05.2003
(51) Int. Cl.: C07D 209/14, A61K 31/404, A61P 43/00

(54) **DERIVES D'INDOLE ET LEUR UTILISATION EN TANT QUE LIGANDS DES RECEPTEURS CB2**
INDOLDERIVATE UND DEREN VERWENDUNG ALS LIGANDEN DER RECEPTOREN CB2
INDOLE DERIVATIVES AND THEIR USE AS LIGANDS FOR CB2 RECEPTORS

(30) Priorité: 17.05.2002 FR 0206133
(43) Date de publication de la demande: 23.02.2005
(73) Titulaire: Sanofi-Aventis, 75013 Paris (FR)
(72) Inventeur: BARTH, Francis, F-34680 Saint-Georges d'Orques (FR); GUILLAUMONT, Carole, Fr-30670 Aigues-Vives (FR); RINALDI-CARMONA, Murielle, F-34680 Saint-Georges d'Orques (FR); VERNHET, Claude, Fr-34270 Le Triadou (FR)
(74) Mandataire: Tsitini-Souleau, Maria
(86) Numéro de dépôt international: PCT/FR2003/001470
(87) Numéro de publication internationale: WO 2003/097597

(56) Documents cités:
- WO-A-01/28557
- WO-A-97/00860

## Description

La présente invention a pour objet de nouveaux composés dérivés d'indole, ligands des récepteurs aux cannabinoïdes CB₂, leur procédé de préparation et les compositions pharmaceutiques en contenant.

Le Δ⁹-THC est le principal constituant actif extrait de *Caruzabis sativa* (Tuner, 1985 ; In Marijuana 1984, Ed. Harvey, DY, IRL Press, Oxford).

De nombreux articles ont décrit non seulement des effets psychotropes des cannabinoïdes mais aussi une influence de ces derniers sur la fonction immunitaire [HOLLISTER L.E. J. Psychoact. Drugs 24 (1992), 159-164]. La plupart des études *in vitro* ont montré des effets immunosuppresseurs des cannabinoïdes : l'inhibition des réponses prolifératives des lymphocytes T et des lymphocytes B induites par les mitogènes [Luo, Y.D. et al., Int. J. Immunopharmacol. (1992) 14, 49-56, Schwartz, H. et al., J. Neuroimmunol. (1994) 55, 107-115], l'inhibition de l'activité des cellules T cytotoxiques [Klein et al., J. Toxicol. Environ. Health (1991) 32, 465-477], l'inhibition de l'activité microbicide des macrophages et de la synthèse du TNFα [Arata, S. et al., Life Sci. (1991) 49, 473-479 ; Fisher-Stenger et al. J. Pharm. Exp. Ther. (1993) 267, 1558-1565], l'inhibition de l'activité cytolytique et de la production de TNFα des grands lymphocytes granulaires [Kusher et al. Cell. Immun. (1994) 154, 99-108]. Dans certaines études, des effets d'amplification ont été observés : augmentation de la bioactivité de l'interleukine-1 par les macrophages résidant de souris ou les lignées cellulaires macrophagiques différenciées, due à des niveaux accrus de TNFα [Zhu et al., J. Pharm. Exp. Ther. (1994) 270, 1334-1339 ; Shivers, S.C. et al. Life Sci. (1994) 54, 1281-1289].

Les effets des cannabinoïdes sont dus à une interaction avec des récepteurs spécifiques de haute affinité, couplés aux protéines G, présents au niveau central (Devane et al., Molecular Pharmacology (1988), 34, 605-613) et périphérique (Nye et al., J. Pharmacol. and Exp. Ther. (1985), 234, 784-791 ; Kaminski et al., Molecular Pharmacol. (1992), 42, 736-742 ; Munro et al., Nature (1993), 365, 61-65).

Les effets centraux des cannabinoïdes relèvent d'un premier type de récepteurs des cannabinoïdes (CB₁) qui est présent principalement dans le cerveau mais aussi à la périphérie. Par ailleurs, Munro et al. [Nature (1993) 365, 61-65] ont cloné un second type de récepteurs des cannabinoïdes, appelé CB₂, qui est présent à la périphérie et plus particulièrement sur les cellules d'origine immune. La présence de récepteurs aux cannabinoïdes CB₂ sur les cellules lymphoïdes peut expliquer l'immunomodulation exercée par les agonistes des récepteurs aux cannabinoïdes évoquée ci-dessus.

Certains dérivés indoliques ont été cités dans l'art antérieur comme présentant une affinité pour les récepteurs CB₂. Ainsi, le brevet US 5 532 237 décrit des composés de formule : 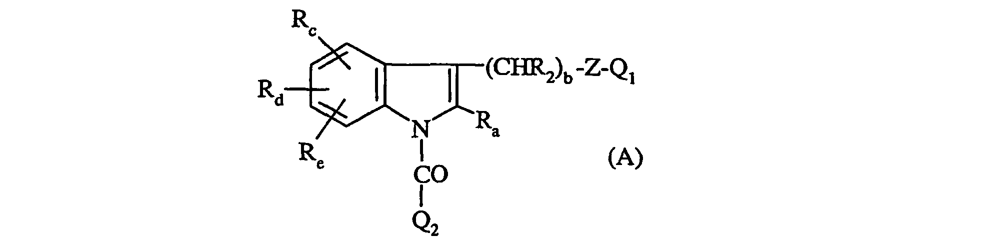 dans laquelle les substituants ont différentes valeurs ;
et la demande de brevet EP 833 818 décrit des composés de formule : 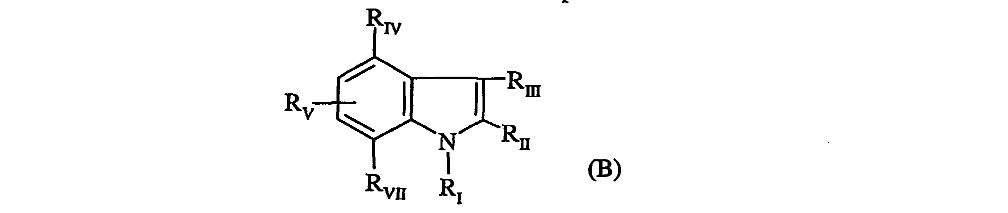 dans laquelle les substituants ont différentes valeurs.

Par ailleurs, le brevet US 4 581354 décrit des dérivés indoliques actifs comme analgésiques et anti-inflammatoires de formule : 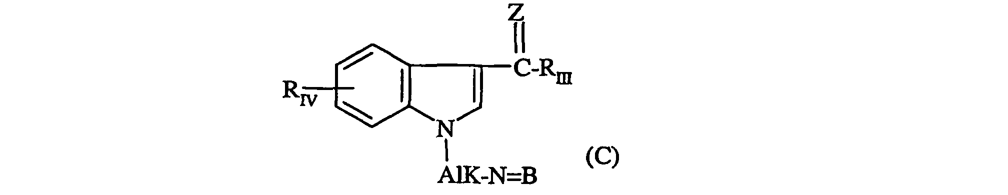 dans laquelle Z peut représenter l'oxygène ou un groupe NOH.

La présente invention a pour objet des composés de formule : 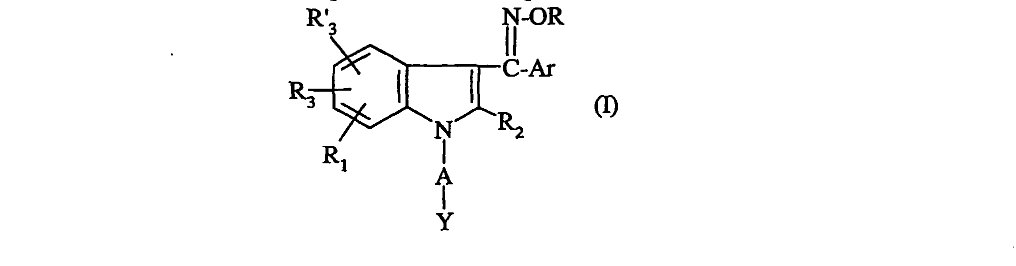 dans laquelle :
- Ar représente :
   a) un phényle mono, di ou trisubstitué par un ou plusieurs groupes choisis parmi : un atome d'halogène, un (C₁-C₄)alkyle, un trifluorométhyle, un amino, un nitro, un hydroxyle, un (C₁-C₄)alcoxy, un (C₁-C₄)alkylsulfanyle, un (C₁-C₄) alkylsulfonyle ;
   b) un naphtyle non substitué ou substitué une ou deux fois par un atome d'halogène, un groupe (C₁-C₄)alkyle ou un trifluorométhyle ;
- A représente un radical alkylène en C₂-C₆ ;
- Y représente un groupe choisi parmi SR₄, SOR₄, SO₂R₄, SO₂NR₅R₆, N(R₇)SO₂R₄, OR₄, NR₇SO₂NR₅R₆ ;
- R représente l'hydrogène, un groupe (C₁-C₄)alkyle ou (C₂-C₄)alcényle ou un groupement (C₂-C₄)alk-NR₈R₉ ;
- R₁, R₃ et R'₃ représentent chacun indépendamment l'un de l'autre l'hydrogène, un atome d'halogène, un groupe hydroxyle, (C₁-C₄)alkyle, trifluorométhyle, (C₁-C₄)alcoxy ;
- R₂ représente l'hydrogène ou un groupe (C₁-C₄)alkyle ;
- R₄ représente un groupe (C₁-C₄)alkyle ou un trifluorométhyle ;
- R₅ et R₆ représentent chacun indépendamment l'hydrogène ou un groupe (C₁-C₄)alkyle ;
- R₇ représente l'hydrogène ou un groupe (C₁-C₄)alkyle ;
- R₈ et R₉ représentent chacun indépendamment l'un de l'autre l'hydrogène ou un groupe (C₁-C₄)alkyle ou R₈ et R₉ ensemble avec l'atome d'azote auquel ils sont liés constituent un radical hétérocyclique comportant de 4 à 7 chaînons et pouvant contenir un autre hétéroatome choisi parmi un atome d'azote, d'oxygène ou de soufre, non substitué ou substitué par un ou plusieurs groupes méthyle ou méthoxy ; ainsi que leurs sels éventuels et leurs solvats.

Par halogène, on entend un atome de chlore, de brome, de fluor ou d'iode.

Par (C₁-C₄)alkyle, on entend un groupe aliphatique en C₁-C₄ linéaire ou ramifié tel que par exemple un groupe méthyle, éthyle, propyle, isopropyle, butyle, isobutyle, tert-butyle.

Par alkylène (alk) en (C₂-C₆) ou respectivement en (C₂-C₄), on entend un groupe linéaire ou ramifié.

Par (C₂-C₄)alcényle, on entend un groupe aliphatique insaturé, en C₂-C₄, linéaire ou ramifié, préférentiellement un groupe allyle.

Lorsque R₈ et R₉ ensemble avec l'atome d'azote auquel ils sont liés, constituent un radical hétérocyclique, celui-ci est préférentiellement choisi parmi : azétidinyle, pyrrolidinyle, pipéridinyle, perhydroazépinyle, pipérazinyle, morpholino, thiomorpholino.

Les composés de formule (I) étant des oximes ou éthers d'oxime, ils existent sous deux formes : syn et anti ; la présente invention comprend chacun des deux isomères et le mélange de ces deux isomères en toutes proportions.

Lorsque les composés de formule (I) comprennent un atome de soufre ou un atome de carbone asymétrique, tous les isomères optiques ainsi que leur mélange en proportions quelconques sont objets de l'invention.

Les sels sont en général préparés avec des acides pharmaceutiquement acceptables mais les sels d'autres acides utiles pour la purification ou l'isolement des composés de formule (I) font également partie de l'invention.

De façon préférentielle, la présente invention est relative aux composés de formule (I) dans laquelle Ar, A, Y, R₁, R₂, R₃, R'₃, R₄, R₅, R₆ et R₇ sont tels que définis ci-dessus et R représente l'hydrogène ou un groupe (C₁-C₄)alkyle.

La présente invention a tout particulièrement pour objet des composés de formule (I) dans laquelle :
- Ar représente :
   a) un phényle mono, di ou trisubstitué par un ou plusieurs groupes choisis parmi : un atome d'halogène, un (C₁-C₄)alkyle, un trifluorométhyle, un amino, un nitro, un (C₁-C₄)alcoxy, un (C₁-C₄)alkylsulfanyle, un (C₁-C₄)alkylsulfonyle ;
   b) un naphtyle non substitué ou substitué une ou deux fois par un atome d'halogène, un (C₁-C₄)alkyle, un trifluorométhyle ;
- A représente un groupe (CH₂)ₙ avec n représente 2, 3 ou 4 ;
   Y représente un groupe choisi parmi SR₄, SOR₄, SO₂R₄, SO₂NR₅R₆, N(R₇)SO₂R₄, OR₄ ;
- R représente l'hydrogène, un groupe (C₁-C₄)alkyle ou (C₂-C₄)alcényle ou un groupement (C₂-C₄)alk-NR₈R₉ ;
- R₁ est en position 7 du noyau indole et représente un atome d'halogène, un groupe (C₁-C₄)alkyle, trifluorométhyle, (C₁-C₄)alcoxy ;
- R₂ représente l'hydrogène ou un groupe (C₁-C₄)alkyle ;
- R₃ représente un atome d'hydrogène ou d'halogène, un groupe (C₁-C₄)alkyle ;
- R'₃ est l'hydrogène ;
- R₄ représente un groupe (C₁-C₄)alkyle ;
- R₅ et R₆ représentent chacun indépendamment l'hydrogène ou un groupe (C₁-C₄)alkyle;
- R₇ représente l'hydrogène ou un groupe (C₁-C₄)alkyle ;
- R₈ et R₉ représentent un (C₁-C₄)alkyle ou ensemble avec l'atome d'azote auquel ils sont liés constituent un radical hétérocyclique choisi parmi : azétidinyle, pyrrolidinyle, pipéridinyle, perhydroazépinyle, pipérazinyle, 4-méthylpipérazin-1-yle, morpholino, thiomorpholino ;
ainsi que leurs sels éventuels et leurs solvats.

On préfère les composés de formule (I) dans laquelle :
- Ar représente un phényle mono ou disubstitué par un atome d'halogène, un groupe méthyle, trifluorométhyle, méthoxy, méthylsulfanyle, méthylsulfonyle ;
- A représente un groupe (CH₂)ₙ avec n représente 2, 3 ou 4 ;
- Y représente un groupe SO₂R₄ ou NHSO₂R₄ ;
- R₁ représente un groupe méthyle, un atome de chlore ou de brome, en position 7 du noyau indole ;
- R₂ représente un groupe méthyle ;
- R₃ est l'hydrogène ou R₃ est en position 6 du noyau indole et représente soit un atome de chlore, soit un groupe méthyle ;
- R'₃ est l'hydrogène ;
- R₄ représente un groupe méthyle ou éthyle ;
- R représente l'hydrogène, un groupe méthyle ou éthyle ou un groupement -(CH₂)₃N(CH₃)₂ ;
ainsi que leurs sels éventuels et leurs solvats.

La présente invention a également pour objet un procédé de préparation des composés de formule (I), de leurs sels éventuels et de leurs solvats.

Ce procédé est caractérisé en ce que l'on traite un dérivé d'aroylindole de formule : 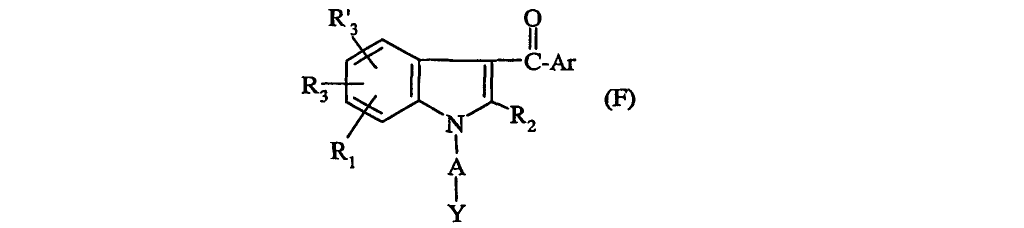 par un dérivé d'hydroxylamine de formule H₂NOR dans laquelle R est tel que défini ci-dessus pour (I).

La réaction est effectuée dans un solvant polaire tel que l'éthanol ou un mélange pyridine-éthanol, à une température comprise entre la température ambiante et la température d'ébullition du solvant.

Le cas échéant, le composé de formule (I) ainsi obtenu est transformé en l'un de ses sels ou solvats.

Le composé obtenu est isolé ; généralement il est constitué d'un mélange des isomères syn et anti de l'oxime ou de l'éther d'oxime selon l'invention.

On peut effectuer la séparation des isomères syn et anti en utilisant un procédé connu de l'homme de l'art, par exemple, la chromatographie préparative.

Selon un procédé de la présente invention, on peut également traiter le composé de formule (F) par l'hydroxylamine (NH₂OH), selon le procédé décrit ci-dessus, et, dans une étape ultérieure, traiter l'oxime ainsi obtenue de formule : 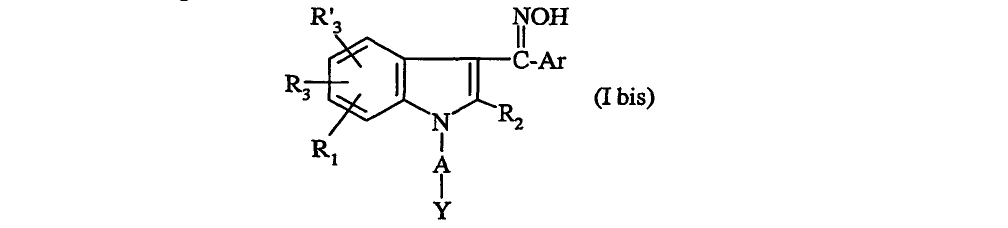 par un halogénure d'alkyle ou d'alcényle de formule RX, dans laquelle X représente un atome d'halogène , ou par un sulfate d'alkyle ou d'alcényle de formule (R)₂SO₄.

La réaction est effectuée en présence d'une base telle que la soude en utilisant un solvant protique comme l'éthanol, ou en présence d'hydrure de sodium dans un solvant oxygéné comme le tétrahydrofurane, à une température comprise entre la température ambiante et la température d'ébullition du solvant.

Les composés de formule (F) peuvent être préparés selon différents procédés décrits ci-après.

Un procédé, appelé procédé A, comprend les étapes suivantes :
a) on traite un indole de formule : 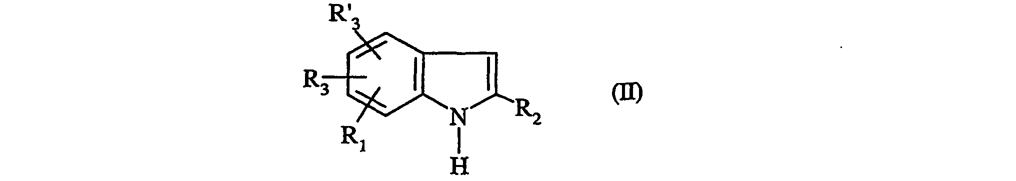 dans laquelle R₁, R₂, R₃ et R'₃ sont tels que définis pour un composé de formule (I), par un halogénure de méthylmagnésium et par un halogénure d'acide de formule ArCOHal (III), dans laquelle Ar est tel que défini pour le composé de formule (I) et Hal représente un atome d'halogène, de préférence le chlore ;
b) on traite le composé ainsi obtenu de formule : 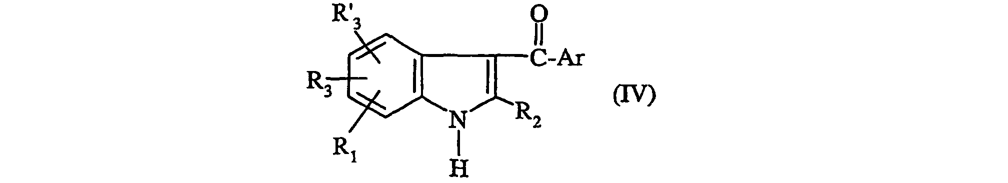 par un halogénure de formule Hal-A-Y (V) dans laquelle -A- et Y sont tels que définis pour un composé de formule (I) et Hal représente un atome d'halogène, de préférence le brome, en présence d'une base.
   A l'étape a) du procédé ci-dessus, l'acylation est effectuée dans un solvant tel que l'éther.
   A l'étape b), on opère en présence d'une base telle que le carbonate de sodium ou le carbonate de potassium, un hydrure tel que l'hydrure de sodium ou un hydroxyde de métal alcalin tel que l'hydroxyde de potassium ; et dans un solvant tel que toluène, DMSO ou DMF, à une température comprise entre la température ambiante et la température d'ébullition du solvant. De façon particulière, lorsque la base utilisée est un hydroxyde de métal alcalin, on peut également effectuer l'étape b) en présence de tris[2-(2-méthoxyéthoxy)éthyl]amine (TDA-1), comme décrit dans Tetrahedron Lett., 1987, 28, 2963 ou d'un sel d'ammonium quaternaire, tel que l'hydrogénosulfate de tétrabutylammonium.
   Il existe une variante du procédé A, appelée procédé A₁, dans lequel l'étape b) du procédé A est modifiée de la façon suivante :
b1) on traite le composé obtenu à l'étape a) de formule : 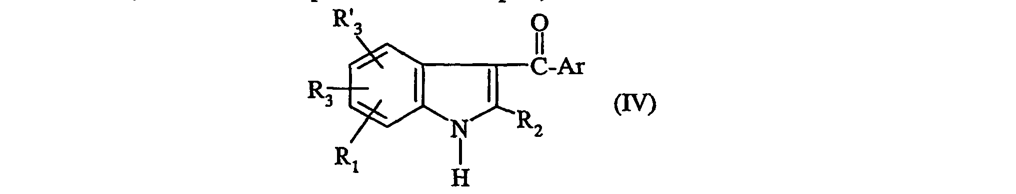 par un composé de formule Z-A-Cl (VI), dans laquelle Z représente soit un groupe hydroxyle soit un atome d'halogène, de préférence le brome ; et -A- est tel que défini pour (I) ;
b2) éventuellement, on traite le composé ainsi obtenu de formule : 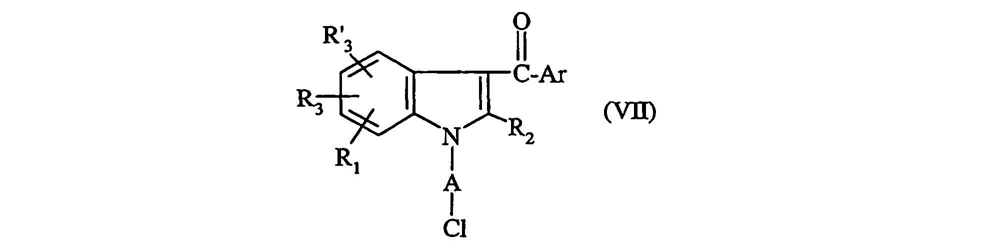 par l'iodure de sodium ;
b3) on traite le composé ainsi obtenu à l'étape b1) de formule (VII) ou à l'étape b2) de formule : 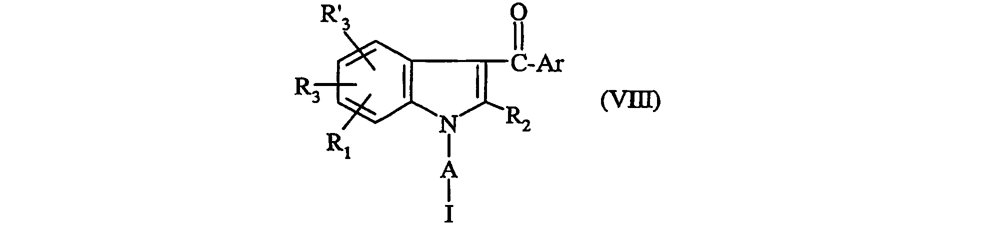 par un anion Y^{⊖}, Y étant tel que défini pour un composé de formule (I) pour former le composé de formule (I).
   Lorsque Z représente un atome d'halogène, l'étape b1) est effectuée en présence d'une base, lorsque Z représente un groupe hydroxyle, l'étape b1) est effectuée en présence de triphénylphosphine et diéthylazodicarboxylate dans un solvant tel que le dichlorométhane.
   A l'étape b2), lorsque celle-ci est réalisée, on utilise un solvant tel que l'acétonitrile, l'acétone ou un autre solvant cétonique.
   Pour effectuer l'étape b3), on utilise un anion obtenu par réaction d'un composé de formule YH (IX) avec NaH dans un solvant tel que le DMF.
   Le procédé A₁ est particulièrement préféré pour préparer des composés de formule (I) dans laquelle Y représente SR₄ ou NHSO₂R₄.
   Selon une autre variante du procédé A, appelée procédé A₂, on peut préparer un composé de formule (I) dans laquelle Y représente un groupe SOR₄ ou un groupe SO₂R₄, à partir d'un composé de formule (I) dans laquelle Y représente un groupe SR₄. Selon ce procédé, après l'étape b) du procédé A ou l'étape b2) ou b3) du procédé A₁, on effectue l'étape supplémentaire suivante :
c1) on traite le composé obtenu de formule : 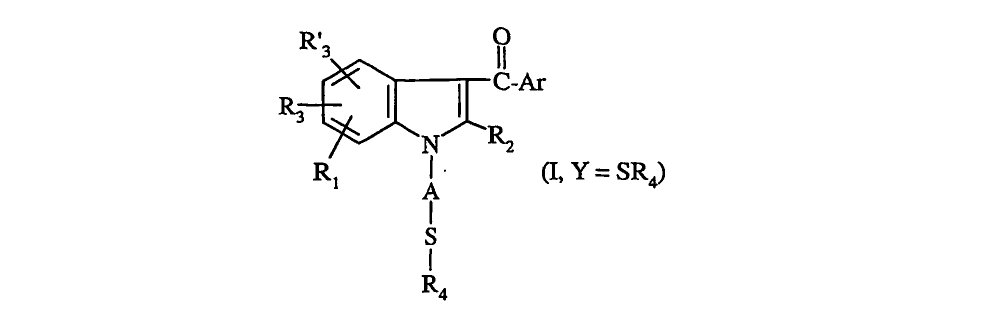 par un agent oxydant.
   Comme agent oxydant, on peut utiliser l'eau oxygénée ou l'acide 3-chloroperbenzoïque ; selon le nombre d'équivalents d'oxydant utilisé et selon la température de la réaction, on obtient un sulfoxyde (I, Y = SOR₄) ou une sulfone (I, Y = SO₂R₄).
   Selon une autre variante du procédé A, appelée procédé A₃, on peut préparer un composé de formule (I) dans laquelle Y représente un groupe N(R₇) SO₂R₄ dans laquelle R₇ est différent de H, à partir d'un composé de formule (I) dans laquelle Y représente un groupe NHSO₂R₄. Selon ce procédé, après l'étape b) du procédé A ou l'étape b2) ou b3) du procédé A₁, on effectue l'étape supplémentaire suivante :
c2) on traite le composé obtenu de formule : 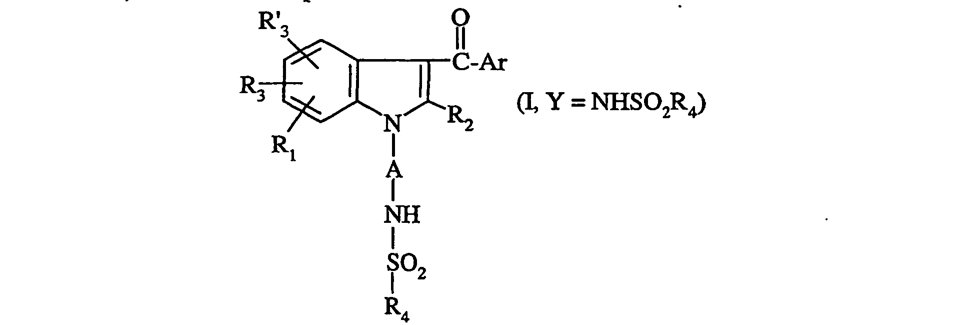 par un agent alkylant, en présence d'une base.
   Comme agent alhylant on utilise, par exemple, un sulfate de dialkyle de formule SO₄(R₇)₂ ou un halogénure d'alkyle de formule R₇Hal, formules dans lesquelles R₇ est tel que défini pour les composés de formule (I) et Hal représente un atome d'halogène, de préférence l'iode, en présence d'une base, telle que l'hydrure de sodium par exemple.
   Selon encore une autre variante du procédé A, appelé procédé A₄, on peut préparer un composé de formule (I) dans laquelle Y représente un groupe SO₂NR₅R₆ à partir d'un composé de formule (I) dans laquelle Y représente un groupe SO₂NHR₅. Selon ce procédé, après l'étape b) du procédé A ou l'étape b2) ou b3) du procédé A₁, on effectue l'étape supplémentaire suivante :
c3) on traite le composé obtenu de formule : 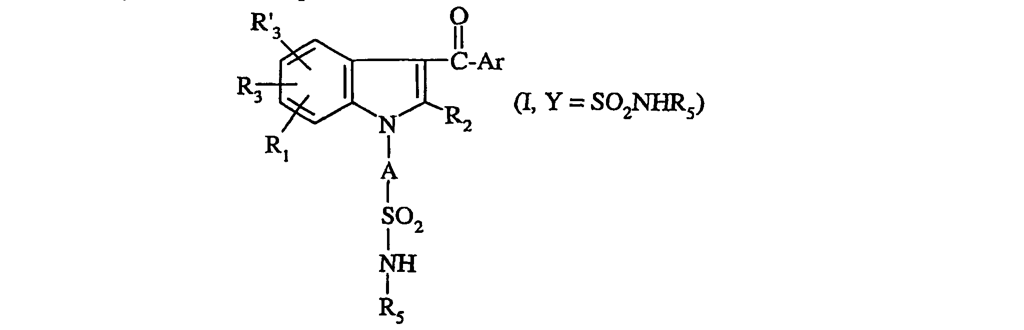 par un agent alkylant, en présence d'une base.
   Comme agent alkylant on utilise, par exemple, un sulfate de dialkyle de formule SO₄(R₆)₂ ou un halogénure d'alkyle de formule R₆Hal, formules dans lesquelles R₆ est tel que défini pour les composés de formule (I) et Hal représente un atome d'halogène, de préférence l'iode, en présence d'une base telle que l'hydrure de sodium.
Lorsque l'on souhaite préparer un composé selon l'invention de formule (I) dans laquelle Y représente un groupe NR₇SO₂R₄ ou un groupe NR₇SO₂NR₅R₆, on peut utiliser une variante du procédé A, appelée procédé A5. Selon ce procédé :
b4) on transforme le composé obtenu à l'étape b1) de formule : 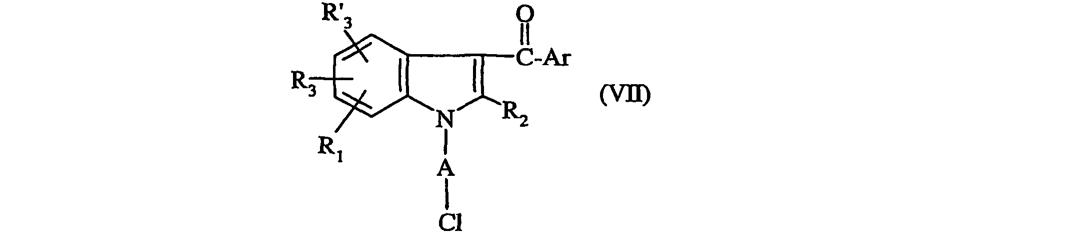 en un composé de formule : 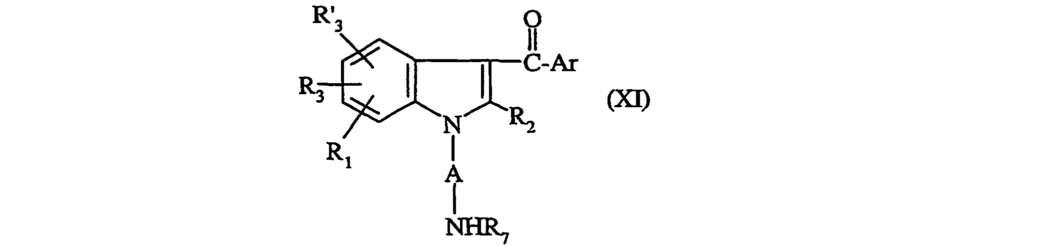 dans laquelle R₇ est tel que défini pour (I) ;
c4) on traite par un halogénure de formule HalSO₂R₄ ou respectivement HalSO₂NR₅R₆ dans laquelle R₄, R₅ et R₆ ont les significations données ci-dessus pour (I).

L'étape b4) peut être réalisée par différents procédés connus de l'homme de l'art, par exemple la réaction de Delépine (Synthesis, 1979, p. 161-179), la réaction de Gabriel (Angew. Chem. Int. Ed. Engl., 1998, 7, 919-930) ou la réaction d'Hebrard (Bull. Soc. Chim. Fr., 1970, 1938).

L'étape c4) peut être réalisée en présence d'une base telle que la triéthylamine.

Selon une méthode alternative du procédé A décrit ci-dessus et de ses variantes, on peut effectuer d'abord l'alkylation de l'azote indolique puis effectuer l'acylation du composé ainsi obtenu. D'après ce procédé alternatif, appelé procédé B :
i) on traite un indole de formule : 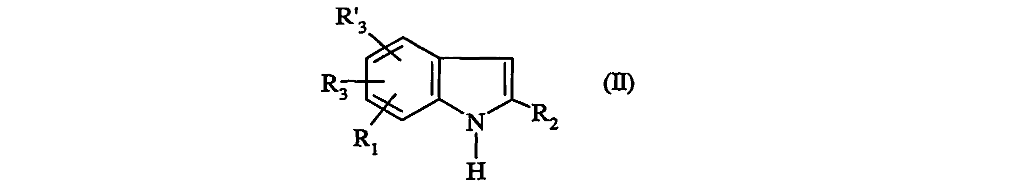 dans laquelle R₁, R₂, R₃ et R'₃ sont tels que définis pour le composé de formule (I) par un halogénure de formule Hal-A-Y (V) dans laquelle -A- et Y sont tels que définis pour un composé de formule (I) et Hal représente un atome d'halogène, de préférence le brome, en présence d'une base ;
ii) on traite le composé ainsi obtenu de formule : 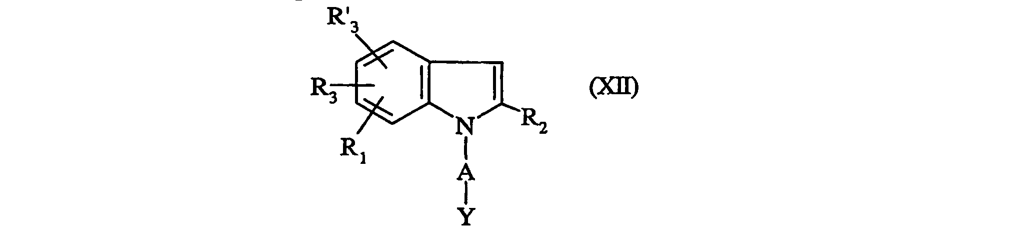 par un halogénure d'acide de formule ArCOHal (III) dans laquelle Ar est tel que défini pour le composé de formule (I) et Hal est un atome d'halogène, de préférence le chlore ou le brome.

L'étape i) du procédé ci-dessus est réalisée dans les conditions décrites pour l'étape b) du procédé A. L'étape ii) est réalisée dans les conditions de Friedel et Crafts, en présence d'un acide de Lewis tel qu'AlCl₃ ou le dichlorure d'éthylaluminium dans un solvant inerte tel que le dichlorométhane ou le dichloroéthane, selon le procédé décrit dans J. Med. Chem., 1995, 38, 3094.

Il existe différentes variantes de l'étape i) du procédé B. Ces variantes correspondent à ce qui a été décrit pour le procédé A.

Selon la variante B₁ du procédé B :
i1) on traite un indole de formule : 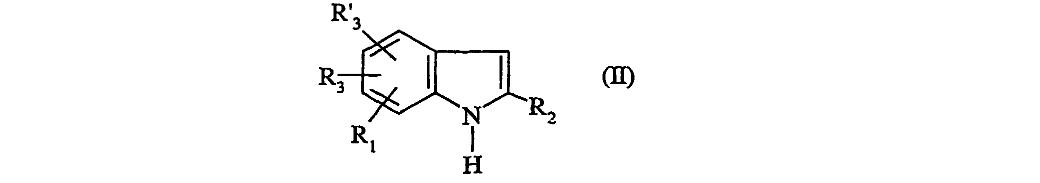 dans laquelle R₁, R₂, R₃ sont tels que définis pour le composé de formule (I), avec un composé de formule Z-A-Cl (VI) dans laquelle -A- est tel que défini pour le composé de formule (I) et Z représente un groupe hydroxyle ou un atome d'halogène, de préférence le brome ;
i2) éventuellement, on traite le composé ainsi obtenu de formule : 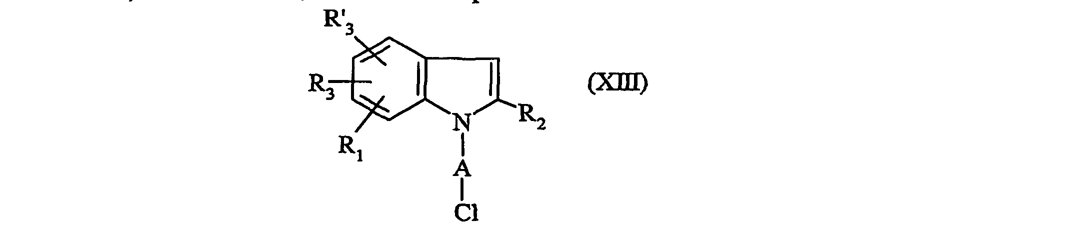 par l'iodure de sodium ;
i3) on traite le composé ainsi obtenu à l'étape i1) ou à l'étape i2) de formule : 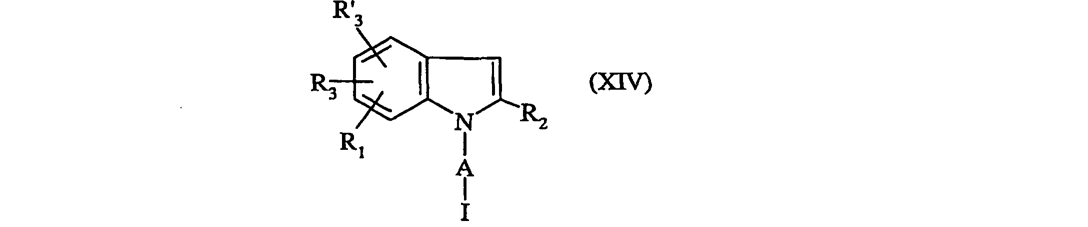 par un anion de formule Y⁻, Y étant tel que défini pour un composé de formule (I) ;
   ii) on traite le composé ainsi obtenu de formule : 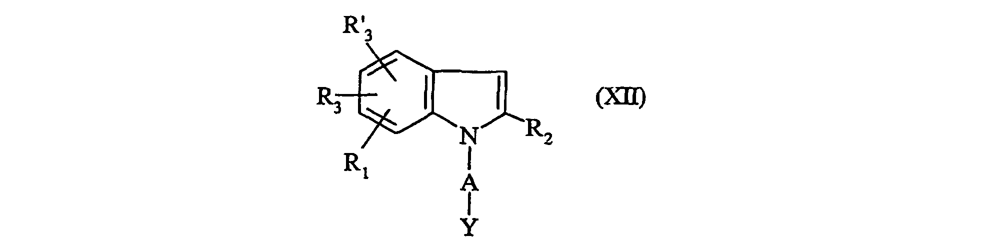 par un halogénure d'acide de formule ArCOHal (III) dans laquelle Ar est tel que défini pour le composé de formule (I) et Hal est un atome d'halogène, de préférence le chlore.

De façon particulière, lorsque l'on souhaite préparer un composé de formule (I) dans laquelle A est (CH₂)₂, on peut utiliser des techniques connues de l'homme de l'art pour introduire la chaîne alkyle de longueur appropriée dans une des étapes, soit de la méthode A, soit de la méthode B.

Les indoles de formule (II) sont connus ou préparés par des méthodes connues telles que décrites dans J. Am. Chem. Soc., 1974, 96, 5495 et 1974, 96, 5512 ou dans Tetrahedron Lett., 1989, 30, 2129.

Les composés selon l'invention ont montré une bonne affinité *in vitro* pour les récepteurs aux cannabinoïdes (CB₂) et une affinité *in vitro* nettement plus faible pour les récepteurs aux cannabinoïdes (CB₁) qu'il s'agisse de récepteurs humains ou de récepteurs de rongeur. Les essais de liaison par affinité (binding) ont été réalisés selon les conditions expérimentales décrites par Devane et al. (Molecular Pharmacology, 1988, 34, 605-613), avec des membranes issues de lignée cellulaires dans lesquelles les récepteurs CB₁ (Matsuda et al., Nature 1990, 346, 561-564) et CB₂ (Munro et al., Nature 1993, 365, 61-65) ont été exprimés. Pour les récepteurs humains, l'affinité *in vitro* aux cannabinoïdes CB₂ exprimé sous forme de Ki (constante d'inhibition) est de l'ordre du nM et le rapport entre l'affinité pour les récepteurs CB₁ et celle pour les récepteurs CB₂ est d'au moins 100.

La nature agoniste ou antagoniste des composés selon l'invention a été démontrée par les résultats obtenus dans les modèles de l'inhibition de l'adénylate-cyclase comme dérit dans M. Rinaldi-Carmona et al., J. Pharmacol. Exp. Ther. 1996, 278, 871-878 et 1998, 284, 644-650.

Les composés selon l'invention possèdent également une affinité *in vivo* pour les récepteurs aux cannabinoïdes présents au niveau de la rate de souris lorsqu'ils sont administrés par voie orale. Les essais ont été réalisés selon les conditions expérimentales décrites par Rinaldi-Carmona et al., J. Pharmacol. Exp. Ther., 1998, 284, 644-650.

Les composés de la présente invention sont notamment des principes actifs de compositions pharmaceutiques dont la toxicité est compatible avec leur utilisation en tant que médicaments.

Selon un de ses aspects, la présente invention concerne l'utilisation d'un composé de formule (I) ou d'un de ses sels ou solvats, pharmaceutiquement acceptable pour la préparation de médicaments destinés à prévenir ou à traiter toute pathologie dans laquelle les récepteurs aux cannabinoïdes CB₂ sont impliqués.

On peut par exemple citer les maladies ou affections suivantes :
les désordres du système immunitaire, notamment les maladies autoimmunes : psoriasis, lupus érythémateux, maladies du tissu conjonctif ou connectivites, syndrome de Sjögrer's, spondylarthrite ankylosante, arthrite rhumatoïde, arthrite réactionnelle, spondylarthrite indifférenciée, maladie de Behcet's, anémies autoimmunes hémolytiques, sclérose en plaques, sclérose latérale amyotrophique, amyloses, le rejet de greffe, les maladies affectant la lignée plasmocytaire ; les maladies allergiques: hypersensibilité retardée ou immédiate, rhinite allergique, dermatite de contact, conjonctivite allergique ; les maladies infectieuses parasitaire, virale ou bactérienne : SIDA, méningites ; l'amylose, les maladies affectant les lignées du système lympho-hématopoîétique ; les maladies inflammatoires, notamment les maladies articulaires : arthrite, arthrite rhumatoïde, ostéoarthrite, spondylite, goutte, vascularite, maladie de Crohn, maladie du colon irritable syndrome de colon irritable (en anglais IBD : inflammatory bowel disease et IBS : irritable bowel syndrome), pancréatite ; l'ostéoporose ; la douleur : les douleurs chroniques de type inflammatoire, les douleurs neuropathiques, les douleurs aiguës périphériques ; les affections oculaires : hypertension oculaire, glaucome ; les affections pulmonaires : maladies des voies respiratoires, asthme, bronchite chronique, obstruction chronique des voies respiratoires (en anglais COPD : chronic obstructive pulmonary disease), emphysème ; les maladies du système nerveux central et les maladies neurogénératives : syndrome de Tourette, maladie de Parkinson, maladie d'Alzheimer, démence sénile, chorée, chorée de Huntington, épilepsie, psychoses, dépression, lésions de la moelle épinière ; la migraine, les vertiges, les vomissements, les nausées en particulier ceux consécutifs à une chimiothérapie ; les maladies cardiovasculaires en particulier hypertension, artériosclérose, crise cardiaque, ischémie cardiaque ; l'ischémie rénale ; les cancers : les tumeurs bégnines de la peau, papillomes et tumeurs cancéreuses, les tumeurs de la prostate, les tumeurs cérébrales (glioblastomes, médullo-epithéliomes, médulloblastomes, neuroblastomes, tumeurs d'origine embryonnaires, astrocytomes, astroblastomes, épendymomes, oligodendrogliomes, tumeur du plexus, neuro-epithéliomes, tumeur de l'épiphyse, épendymoblastomes, neuroectodermique, méningiomes malins, sarcomatoses, mélanomes malins, schwannomes) ; les maladies gastro-intestinales ; l'obésité ; le diabète.

L'utilisation des composés selon l'invention pour la prévention et/ou le traitement des maladies ci-dessus mentionnées, ainsi que pour la préparation de médicaments destinés à traiter ces maladies fait partie intégrante de l'invention.

Les composés de formule (I) ci-dessus, ou l'un de leurs sels ou solvats pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,1 à 4000 mg par jour, plus particulièrement de 0,5 à 1000 mg selon l'âge du sujet à traiter ou le type de traitement : prophylactique ou curatif.

Ainsi, selon un autre de ses aspects, la présente invention concerne des compositions pharmaceutiques renfermant, en tant que principe actif, un composé de formule (I), ou l'un de ses sels ou solvats pharmaceutiquement acceptables ainsi qu'un ou plusieurs excipients pharmaceutiquement acceptables.

Dans les compositions pharmaceutiques de la présente invention pour l'administration par voie orale, sublinguale, inhalée, sous-cutanée, intramusculaire, intraveineuse, topique, locale, intratrachéale, intranasale, transdermique, locale ou rectale, les principes actifs peuvent être administrés sous formes unitaires d'administration, en mélange avec des supports pharmaceutiques classiques, aux animaux et aux êtres humains.

Les formes unitaires d'administration appropriées comprennent les formes par voie orale telles que les comprimés, les gélules molles ou dures, les poudres, les granules, les solutions ou suspensions orales, les formes d'administration sublinguale, buccale, intratrachéale, intraoculaire, intranasale, par inhalation, les aérosols, les formes d'administration topique, transdermique, les implants, les formes d'administration sous-cutanée, intramusculaire, intraveineuse et les formes d'administration rectale.

Pour l'administration topique on peut utiliser les composés selon l'invention dans des crèmes, des pommades, des gels, ou des lotions.

Dans chaque unité de dosage le principe actif de formule (I) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,1 à 1000 mg de principe actif, de préférence de 0,5 à 250 mg devant être administrés une à quatre fois par jour.

Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Les composés selon l'invention pourront également être utilisés pour la préparation de compositions à usage vétérinaire.

De plus, les composés selon l'invention, tel quel ou sous forme radiomarquée peuvent être utilisés comme outils pharmacologiques chez l'homme ou chez l'animal, pour la détection et la marquage des récepteurs aux cannabinoïdes CB₂.

Les PREPARATIONS et EXEMPLES suivants illustrent l'invention sans toute fois la limiter.

Dans les Préparations et dans les Exemples on utilise les abréviations suivantes :
éther : éther diéthylique
éther iso : éther diisopropylique
DMF : N,N-diméthylformamide
THF : tétrahydrofurane
DCM : dichlorométhane
AcOEt : acétate d'éthyle
TDA-1 : tris[2-(2-méthoxyéthoxy)éthyl]amine

Ether chlorhydrique : solution saturée d'acide chlorhydrique dans l'éther diéthylique
Triton B : N-benzyltriméthylammonium hydroxyde
F : point de fusion
TA : température ambiante
Eb : température d'ébullition

Les spectres de résonance magnétique du proton (RMN ¹H) sont enregistrés à 200 MHz dans du DMSO-d₆, en utilisant le pic du DMSO-d₆ comme référence. Les déplacements chimiques δ sont exprimés en parties par million (ppm). Les signaux observés sont exprimés ainsi : s: singulet ; se : singulet élargi ; d : doublet ; d.d : doublet dédoublé ; t : triplet ; td : triplet dédoublé ; te : triplet élargi ; q : quadruplet ; qe : quadruplet élargi ; qt : quintuplet ; m : massif ; mt : multiplet ; sp : septuplet.

### Préparation des indoles de formule (II).

### Préparation 1.1

### 2-méthyl-7-chloro-1H-indole.

Sous atmosphère d'azote, on place 42,1 g de 2-chloronitrobenzène dans 850 ml de THF. On refroidit à -40°C puis on ajoute goutte à goutte, 1,6 l de bromure d'isopropènylmagnésium 0,5M dans le THF. Après 1 heure à -40°C sous agitation, on hydrolyse par 400 ml d'une solution saturée de NH₄Cl. La phase aqueuse est extraite 2 fois à l'éther. On sèche puis évapore la phase organique et le résidu est chromatographié sur silice en éluant par du toluène. On obtient 20,3 g du composé attendu.

RMN : δ (ppm) : 2,4 : s : 3H ; 6,2 : s : 1H ; 6,9 : t : 1H ; 7,1 : d : 1H ; 7,4 : d : 1H ; 11,2 : se : 1H.

### Préparation 1.2

### 7-Bromo-2-méthyl-1H-indole.

On place 27,0 g de 2-bromonitrobenzène dans 400 ml de THF. On place le milieu sous azote et on refroidit à -55°C puis on ajoute goutte à goutte, 800 ml de bromure d'isopropénylmagnésium 0,5 M dans le THF. On laisse sous agitation pendant 1 heure puis on verse le milieu dans une solution saturée de NH₄Cl. On extrait à l'éther, évapore puis reprend au DCM. On lave par une solution saturée de NaCl. On sèche et évapore puis le résidu est chromatographié sur silice en éluant par un mélange AcOEt/cyclohexane (1/9 ; v/v). On obtient 10,7 g du composé attendu.

RMN : δ (ppm) : 2,4 : s : 3H ; 6,2 : s : 1H ; 6,9 : t : 1H ; 7,2 : d : 1H ; 7,4 : d : 1H ; 11,2 : se : 1H.

### Préparation 1.3

### 6,7-dichloro-2-méthyl-1H-indole.

On introduit sous azote 1600 ml de bromure d'isopropénylmagnésium 0,5 M dans le THF, on refroidit à -20°C et ajoute 51,2 g de 2,3-dichloronitrobenzène dans 250 ml de THF anhydre puis on laisse 1 heure sous agitation à -20°C. On verse le milieu réactionnel à -20°C sur 1 litre de solution saturée de NH₄Cl, on dilue avec Et₂O puis on lave 2 fois la phase aqueuse avec Et₂O. On réunit les phases organiques que l'on concentre à sec. On extrait au DCM, lave 2 fois à l'eau puis par une solution saturée de NaCl. On sèche puis évapore et on chromatographie le résidu sur un mélange hexane/AcOEt (95/5 ; v/v). On obtient 24,27 g du composé attendu, F = 70-71°C

Ainsi, on a préparé les dérivés d'indole décrits dans le tableau 1 ci-après :

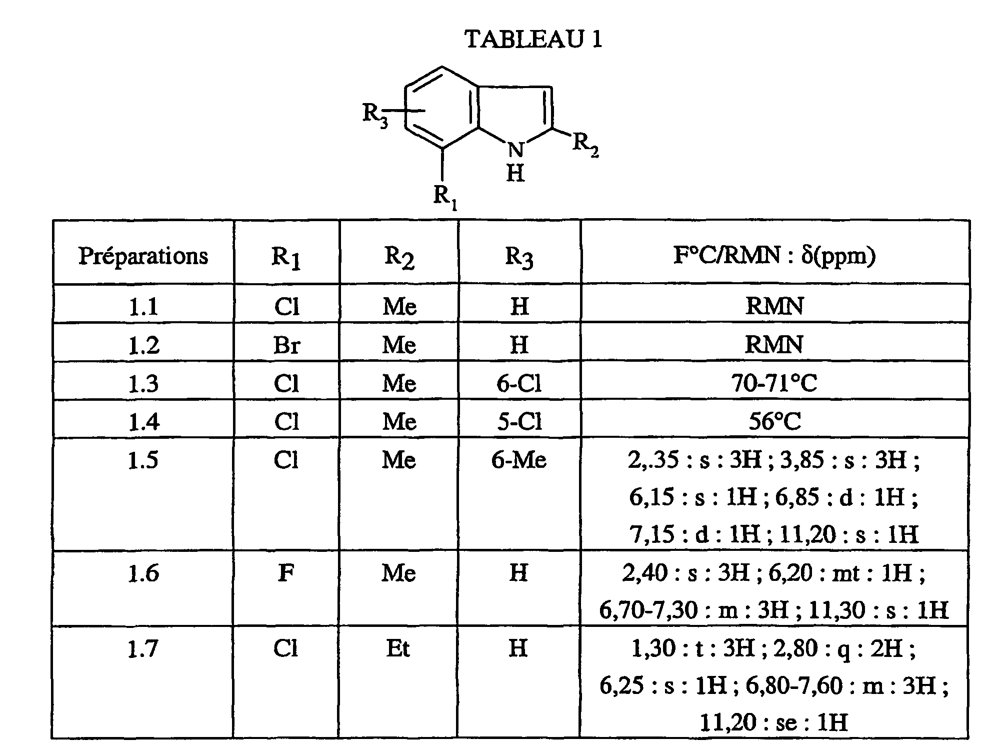

En utilisant le procédé A, on prépare les intermédiaires de formule (IV) décrits ci-après :

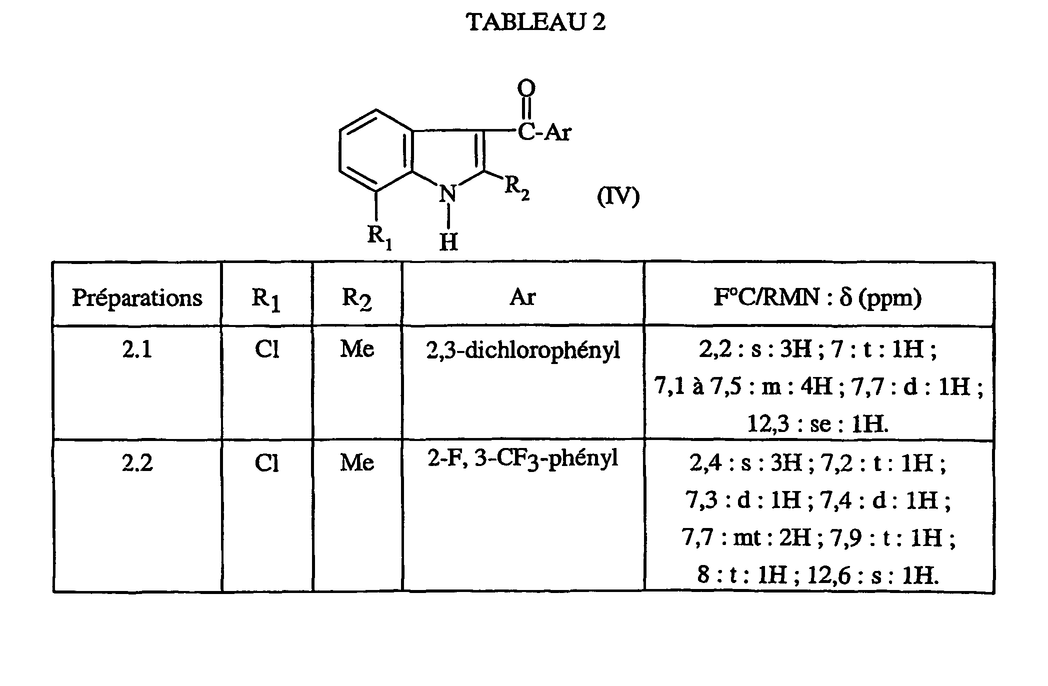

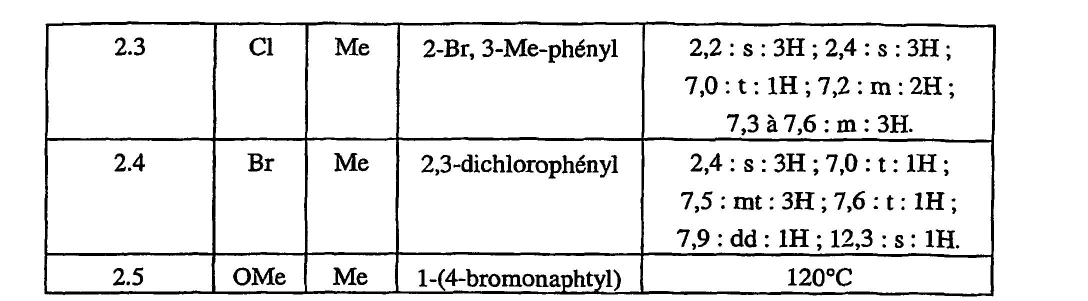

En utilisant le procédé B ou le procédé B₁, on prépare les intermédiaires de formule (XII) décrits ci-après :

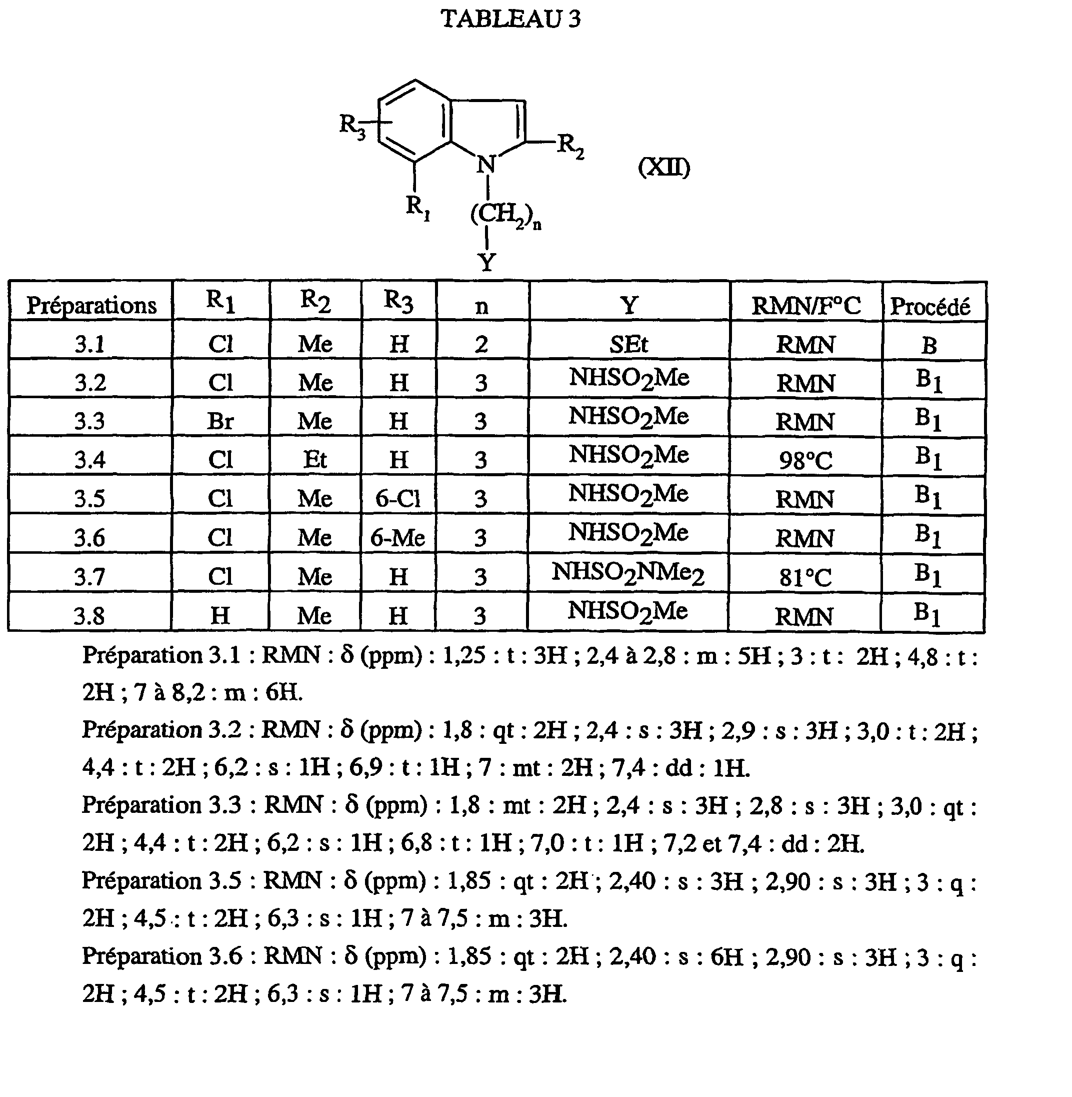

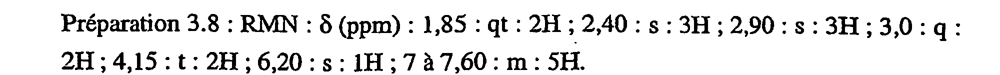

### Préparation 4.1 (Procédé B₁)

### N-(3-(7-Chloro-3-(2-fluoro-3-trifluorométhylphényl)-2-méthyl-1H-indol-1-yl)propyl)méthanesulfonamide.

(F) : R₁ = Cl, R₂ = Me, R₃ = R'₃ = H, Ar = 2-fluoro-3-trifluorométhylphényle, Y = NHSO₂Me, A = (CH₂)ₙ, n = 3.

### A) 7-chloro-1-chloropropyl-2-méthyl-1H-indole.

Sous azote, on place 40 g de 7-chloro-2-méthyl-1*H*-indole dans 60 ml de toluène avec 2,8 g de KOH. Après 30 minutes sous agitation à TA, on ajoute 7,7 g de 3-chloro-1-bromopropane puis on chauffe à reflux pendant 3 heures. Le milieu est extrait à l'éther. La phase organique est lavée à l'eau, par une solution d'HCl à 10 %, à l'eau, par une solution saturée de NaCl. On sèche, on évapore et on obtient 6,19 g du composé attendu.

### B) N-(3-(7-Chloro-2-méthyl-1H-indol-1-yl)propyl)méthanesulfonamide

Sous azote, on prépare un mélange contenant 2,2 g de NaH à 60 % dans l'huile et 170 ml de DMF que l'on refroidit à 0°C. On ajoute 4,0 g de NH₂SO₂CH₃ puis on laisse revenir à TA et on ajoute 5,0 g du composé de l'étape précédente. On chauffe à 130°C pendant 6 heures. Le milieu est extrait au DCM, la phase organique est lavée à l'eau puis par une solution de NaCl saturée. On sèche, on évapore, puis le résidu est chromatographié sur silice en éluant par un mélange cyclohexane/AcOEt (30/70 ; v/v). On obtient 1,92 g du composé attendu.

### C) N-(3-(7-Chloro-3-(2-fluoro-3-trifluorométhylphényl)-2-méthyl-1H-indol-1-yl)propyl)méthanesulfonamide.

Sous azote, on mélange 0,80 g du composé de l'étape précédente, 0,90 g de chlorure de 2-fluoro-3-trifluorométhylbenzoyle dans 60 ml de DCM. On abaisse la température à 0°C puis on ajoute 34 ml de dichloroéthylaluminium, 1,8 M dans le toluène. On laisse revenir à TA puis on agite le milieu pendant 15 heures. On extrait au DCM. La phase organique est lavée à l'eau, par une solution saturée de NaCl. On sèche, on évapore. Le produit est cristallisé dans un mélange DCM-éther. On obtient 550 mg du composé attendu, F = 168°C.

RMN : δ (ppm) : 2 : mt : 2H ; 2,4 : s : 3H ; 3,0 : s : 3H ; 3,2 : mt : 2H ; 4,7 : mt : 2H ; 7 à 7,5 : m : 3H ; 7,7 : t : 1H ; 8 : t : 1H ; 8,2 : t : 1H.

### Préparation 4.2

### 7-Bromo-2-méthyl-1-(3-(méthylsulfinyl)propyl)-1H-indol-3-yl)-(2,3-dichlorophényl)méthanone.

(F) : R₁ = Br, R₂ = Me, R₃ = R'₃ = H, Ar = 2,3-dichlorophényle, Y = -SOMe, A = (CH₂)ₙ, n = 3.

### A) (7-Bromo-2-méthyl-1H-indol-3-yl)-(2,3-dichlorophénylméthanone).

On place 10,7 g de 7-bromo-2-méthyl-1*H*-indole dans 100 ml de THF et on refroidit à -10°C. A cette température, on ajoute 22 ml de bromure de méthylmagnésium 3M dans l'éther. On laisse revenir à TA puis on refroidit à -5°C et on ajoute goutte à goutte, 13,5 g de chlorure de 2,3-dichlorobenzoyle dissous dans 80 ml de THF. On laisse revenir à TA puis on verse le milieu sur une solution saturée de NH₄Cl. On extrait à l'éther, puis on lave la phase organique par une solution de NaOH à 10 %, de l'eau, une solution saturée de NaCl. On sèche et évapore, puis le résidu est chromatographié sur silice en éluant par un mélange AcOEt/cyclohexane (10/90 ; v/v). Le produit obtenu cristallise dans l'éther, on obtient 5 g du composé attendu.

### B) (7-Bromo-2-méthyl-1-(3-chloropropyl)-1H-indol-3-yl)-(2,3-dichlorophényle) méthanone.

A 1 g de potasse pilée, on ajoute 3 g du composé de l'étape précédente, 0,3 g de TDA-1 et 100 ml de toluène puis on chauffe à reflux pendant 30 minutes et on ajoute 5 g de 1-bromo-3-chloropropane. On laisse refroidir le milieu à TA puis on le verse sur 100 ml d'une solution d'HCl à 10 %. Le milieu est extrait au toluène puis la phase organique est lavée à l'eau puis par une solution saturée de NaCl. On sèche et évapore puis on chromatographie le résidu sur silice en éluant au DCM. On obtient 3,25 g du composé attendu, F = 139°C.

### C) (7-Bromo-2-méthyl-1-(3-(méthylsulfanyl)propyl)-1H-indol-3-yl)-(2,3-dichlorophénylméthanone).

A TA, on mélange 3 g du composé de l'étape précédente et 0,62 g de MeSNa dans 40 ml d'éthanol. On chauffe à reflux pendant 2 heures et demie puis on laisse refroidir. On verse le milieu sur une solution de soude à 10 %. Le milieu est extrait à l'éther puis la phase organique est lavée par une solution saturée de NaCl. On obtient 2 g du composé attendu, F = 119°C.

RMN : δ (ppm) : 2 : mt : 2H ; 2,1 : s : 3H ; 2,4 : s : 3H ; 2,6 : t : 2H ; 4,6 : t : 2H ; 7 : t : 1H ; 7,4 à 7,6 : m : 4H ; 7,8 : dd: 1H.

### D) 7-Bromo-2-méthyl-1-(3-(méthylsulfinyl)propyl)-1H-indol-3-yl)-(2,3-dichlorophényl)méthanone.

On place 2,5 g du composé de l'étape précédente dans 50 ml d'acide acétique et on refroidit à 10°C. On ajoute 0,8 ml d'H₂O₂ sous agitation puis on laisse revenir à TA et on maintient l'agitation pendant 1 heure et demie. On évapore puis on extrait le milieu par AcOEt. La phase organique est lavée par une solution de NaOH à 10 %, de l'eau, une solution saturée de NaCl. On évapore, le produit obtenu est recristallisé dans un mélange AcOEt/MeOH (9/1 ; v/v). On obtient 1,1 g du composé attendu, F = 137°C.

RMN : δ (ppm) : 2,1 : qt : 2H ; 2,4 et 2,6 : 2s : 6H ; 2,8 à 3,2 : mt : 2H ; 4,8 : t : 2H ; 7,1 : t : 1H ; 7,5 : m : 4H ; 7,9 : d : 1H.

### Préparation 4.3 (procédé B)

### N-(3-(6,7-dichloro-3-[2-fluoro-3-(trifluorométhyl)benzoyl]-2-méthyl-1H-indol-1-yl)propyl)méthanesulfonamide.

(F) : R₁ =R₃ =Cl, R₂ = Me, R'₃ = H, Ar = 2F-3-CF₃-phényle, Y = -NHSO₂Me, A = (CH₂)ₙ, n = 3.

### A) 6,7-Dichloro-1-(3-chloropropyl)-2-méthyl-1H-indole.

Sous azote, on introduit 7,84 g de soude pilée, 190 ml de toluène, 7 g de 6,7-dichloro-2-méthyl-1*H*-indole, 85 ml de toluène et 0,7 g de tétrabutylammonium hydrogénosulfate. On chauffe à reflux pendant 30 minutes puis on ajoute 14 ml de 1-bromo-3-chloropropane et on maintient le reflux pendant 2 heures. On verse le milieu réactionnel sur de l'eau, on lave la phase aqueuse par du toluène. On extrait au toluène puis on lave à l'eau et par une solution saturée de NaCl,. On sèche et évapore pour obtenir 11,3 g du composé attendu.

### B) 6,7-Dichloro-1-(3-iodopropyl)-2-méthyl-1H-indole.

On introduit 11,3 g du composé de l'étape précédente dans 520 ml d'acétonitrile et 43 g de NaI puis on chauffe au reflux pendant 3 jours. On verse le milieu réactionnel sur de l'eau, on le dilue avec du toluène puis on lave 2 fois la phase aqueuse avec du toluène. Les phases organiques sont réunies puis on lave à l'eau puis par une solution saturée de NaCl. On sèche et concentre pour obtenir 13,93 g du composé attendu.

### C) N-[3-(6,7-dichloro-2-méthyl-1H-indol-1-yl)propyl]méthanesulfonamide.

Sous azote, on introduit 6,04 g de NaH à 60 % dans 400 ml de DMF anhydre. On refroidit à 5°C puis on ajoute 14,35 g de méthanesulfonamide dans 200 ml de DMF anhydre. Après 10 minutes sous agitation à 5°C, on ajoute 13,9 g du composé obtenu à l'étape précédente dans 200 ml de DMF anhydre et on laisse revenir à TA. Après 3 heures sous agitation, on verse le milieu réactionnel sur de l'eau puis on dilue par du DCM. On lave 3 fois la phase aqueuse avec du DCM puis on réunit les phases organiques. On lave à l'eau et par une solution saturée de NaCl. On sèche et concentre puis le résidu est chromatographié sur silice en éluant par un mélange cyclohexane/AcOEt (50/50 ; v/v). On obtient 7,43 g du composé attendu.

RMN : δ (ppm) : 1,80 : mt : 2H ; 2,35 : s : 3H ; 2,60 : s : 6H ; 2,90 : mt : 2H ; 4,40 : t : 2H ; 6,30 : s : 1H ; 7,10 : d : 1H ; 7,20 : mt : 1H ; 7,35 : d : 1H.

### D) N-(3-(6,7-dichloro-3-[2-fluoro-3-(trifluorométhyl)benzoyl]-2-méthyl-1H-indol-1-yl)propyl)méthanesulfonamide

On introduit 1 g du composé de l'étape précédente et 1,35 g de chlorure de 2-fluoro-3-(trifluorométhyl)benzoyle dans 120 ml de DCM. On refroidit entre -20°C et - 25°C et on ajoute à l'aide d'une seringue 3,3 ml de dichloroéthylaluminium. On laisse revenir à température ambiante et on maintient 3 heures sous agitation. On verse le milieu réactionnel sur de l'eau, on lave 3 fois la phase aqueuse avec du DCM puis on réunit les phases organiques que l'on filtre sur Célite®. On lave par une solution de NaOH à 10 %, par de l'eau, une solution de HCl à 10 % puis une solution saturée de NaCl. On obtient 0,94 g du composé attendu qui cristallise dans l'éther, F = 181°C.

En opérant selon l'un des procédés décrits, on a préparé les composés de formule II rassemblés dans le tableau ci-après :

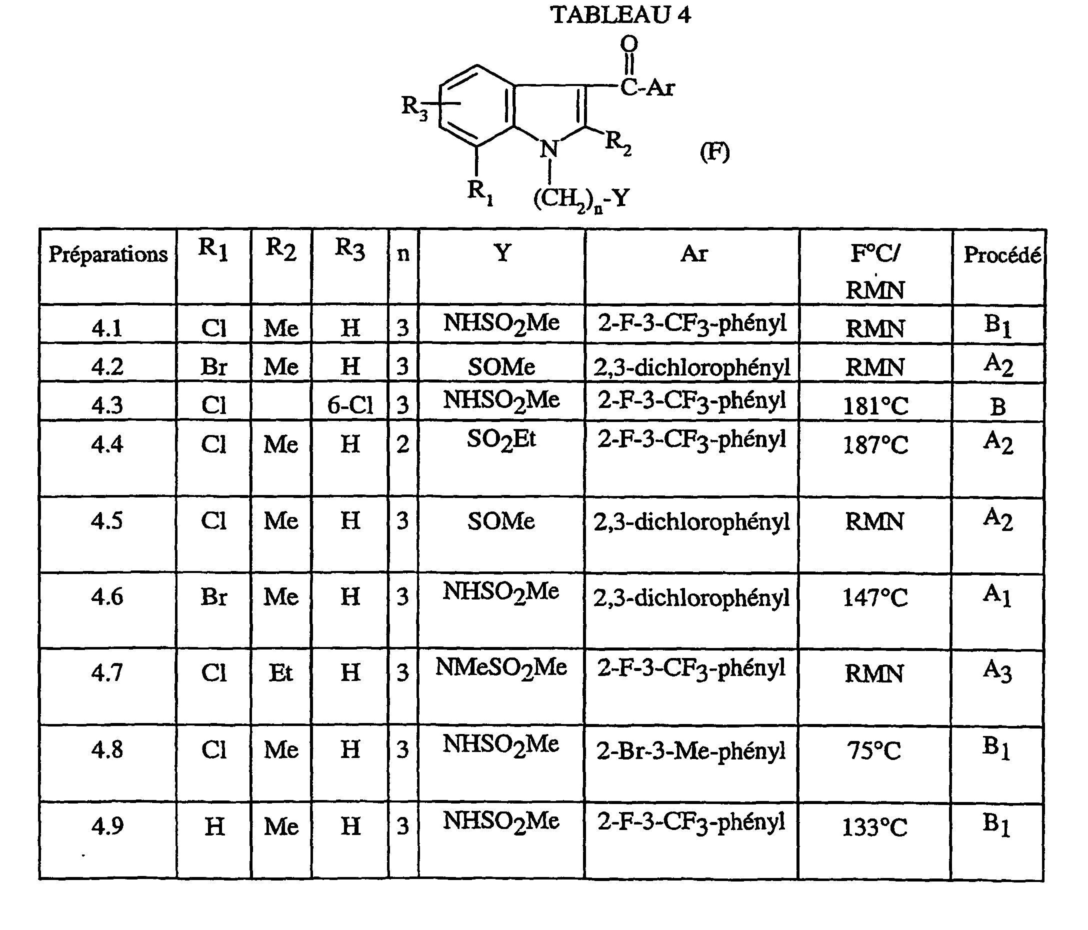

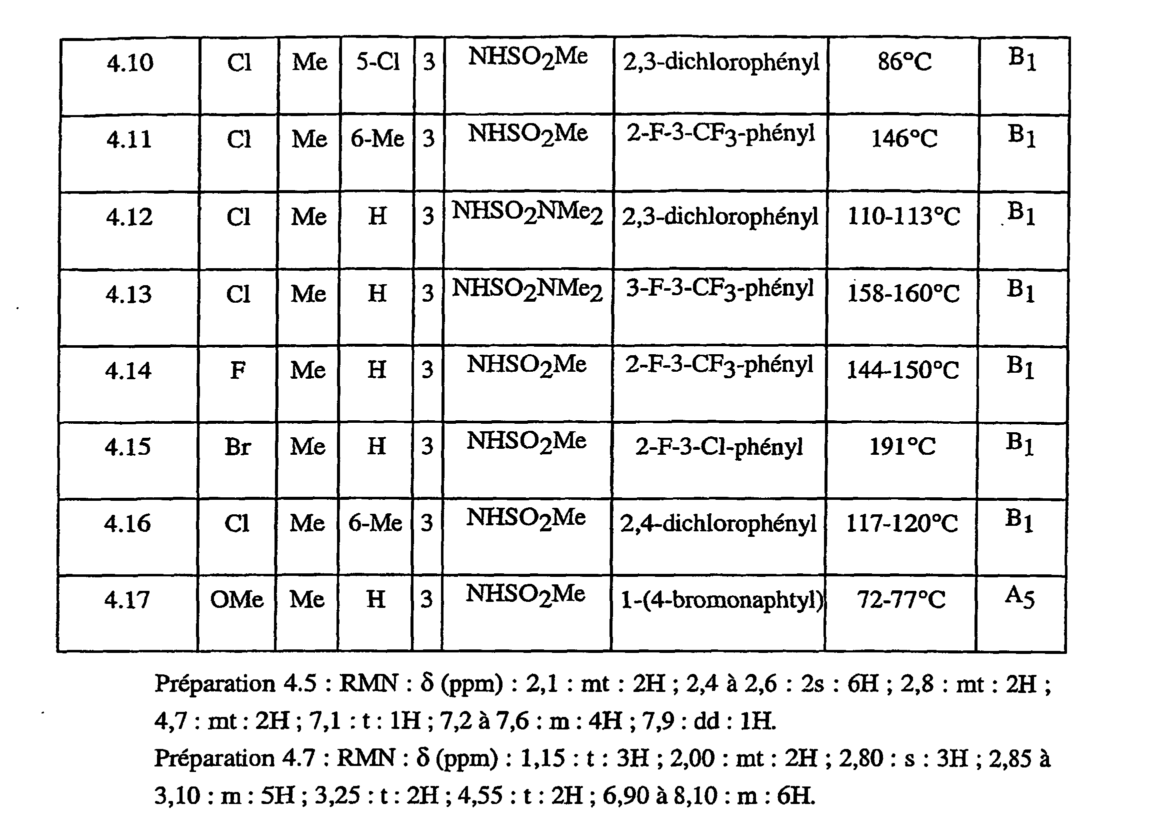

### EXEMPLE 1 : Composé 1

### (I) : R₁ = 7-Cl, R₂ = Me, R₃ = R'₃ = H, Ar = 2-fluoro-3-trifluorométhylphényle, Y = SO₂Me, A = (CH₂)₃, R= H.

### N-(3-(7-Chloro-3-((2-fluoro-3-trifluorométhyl)-phényl)-(hydroxyimino)méthyl-2-méthyl-1H-indol-1-yl)propyl)méthanesulfonamide.

On place sous azote 4,45 g du composé de la préparation 4.1 et 6,3 g d'hydroxylamine dans un mélange de 25 ml de pyridine et 26 ml d'éthanol et on chauffe à reflux une nuit.

On concentre à sec puis on reprend par un mélange éther/solution aqueuse d'HCl 2N, on lave la phase organique par de l'eau (2 fois) puis par une solution saturée de NaCl.

On sèche sur MgSO₄ et on évapore puis on chromatographie sur silice en éluant par un mélange DCM/MeOH (100/1 à 97/3 ; v/v).

On obtient 2,72 g du composé attendu, F = 172°C.

### EXEMPLE 2 : Composé 2

### (I) : R₁ = 7-Cl, R₂ = Me, R₃ = R'₃ = H, Ar = 2-fluoro-3-trifluorométhylphényle, Y = SO₂Me, A = (CH₂)₃, R= Me.

### N-(3-(7-Chloro-3-((2-fluoro-3-trifluorométhyl)-phényl)-(méthoxyimino)méthyl-2-méthyl-1H-indol-1-yl)propyl)méthanesulfonamide.

On place sous azote 1 g du composé de la préparation 4.1 et 1,70 g de O-méthylhydroxylamine dans un mélange de 7,5 ml de pyridine et 11 ml d'éthanol et on chauffe à reflux pendant 3 jours. On concentre à sec puis on reprend dans un mélange éther/solution aqueuse d'acide chlorhydrique à 10 %.

On lave la phase aqueuse 2 fois par de l'éther, on réunit les phases éthérées puis on les lave par de l'eau, et par une solution saturée de NaCl.

On sèche sur MgSO₄, puis on chromatographie sur silice en éluant par un mélange de cyclohexane/AcOEt (50/50 ; v/v). On obtient 1,06 g du composé attendu sous forme d'un solide vitreux, F = 62-65°C.

### EXEMPLE 3 : Composé 28

### Chlorhydrate de N-(3-(7-chloro-3-(((3-diméthyamino)propoxyimino)-(fluoro-3-trifluorométhyl)phényl)méthyl)-2-méthyl-1H-indol-1-yl)propyl)méthanesulfonamide.

### A) Acétone O-(3-(diméthylamino)propyl)oxime.

Sous azote, on introduit 1 litre d'éthanol puis 46 g de sodium coupé en morceaux et on refroidit à 20°C. On ajoute 73,1 g d'acétooxime et 158 g de chlorhydrate de 3-chlorodiméthylaminopropane et on chauffe à reflux pendant 2 heures. Après une nuit sous agitation à TA, on filtre le NaCl formé puis on acidifie par 120 ml d'HCl concentré et on concentre à sec. On reprend par 100 ml d'eau puis on ajoute à 10°C 160 ml de soude concentrée. On extrait 2 fois à l'éther puis on sèche sur MgSO₄. L'éther est distillé à pression atmosphérique puis le produit est distillé sous une pression de 18 mm de mercure = 2400 pascals. On obtient 74,1 g du composé attendu.

### B) Dichlorhydrate de 3-(aminooxy)propyldiméthylamine.

On chauffe à reflux pendant 1 nuit un mélange contenant 74,1 g du composé de l'étape précédente dans 330 mg d'eau et 140 ml d'HCl concentré. On distille l'acétone formée et on poursuit le reflux pendant 1 heure. On concentre à sec, ajoute de l'éthanol et du toluène puis concentre à nouveau à sec. On reprend par 200 ml d'isopropanol, ajoute 200 ml d'éther puis 200 ml d'acétonitrile. Après 1 heure sous agitation, on filtre le solide formé et on le rince à l'acétonitrile contenant 20 % de d'isopropanol puis à l'éther ; on sèche sous vide, sur P₂O₅ pour obtenir 75 g du composé attendu, F = 158°C.

### C) Chlorhydrate de N-(3-(7-chloro-3-(((3-diméthyamino)propoxyimino)-(fluoro-3-trifluorométhyl)phényl)méthyl)-2-méthyl-1H-indol-1-yl)propyl)méthanesulfonamide.

On place, sous azote, 0,79 g du composé de la Préparation 4.1 et 3 g de dichlorhydrate de 3-(aminooxy)propyldiméthylamine dans 40 ml d'éthanol et on laisse sous agitation à reflux pendant 6 heures. On concentre à sec puis on reprend dans un mélange acétate d'éthyle/solution aqueuse d'HCl 2N. On lave la phase organique par une solution saturée de NaCl puis on sèche sur MgSO₄ et évapore. On chromatographie sur silice en éluant par un mélange DCM/MeOH (95/5 ; v/v). On obtient 280 mg du composé attendu, F = 68°C.

En procédant comme décrit dans les exemples ci-dessus, on prépare les composés selon l'invention rassemblés dans le tableau ci-après.

Les composés sont caractérisés par leur point de fusion ou leur spectre RMN, dans tous les cas on a vérifié que le spectre RMN est compatible avec la structure du composé.

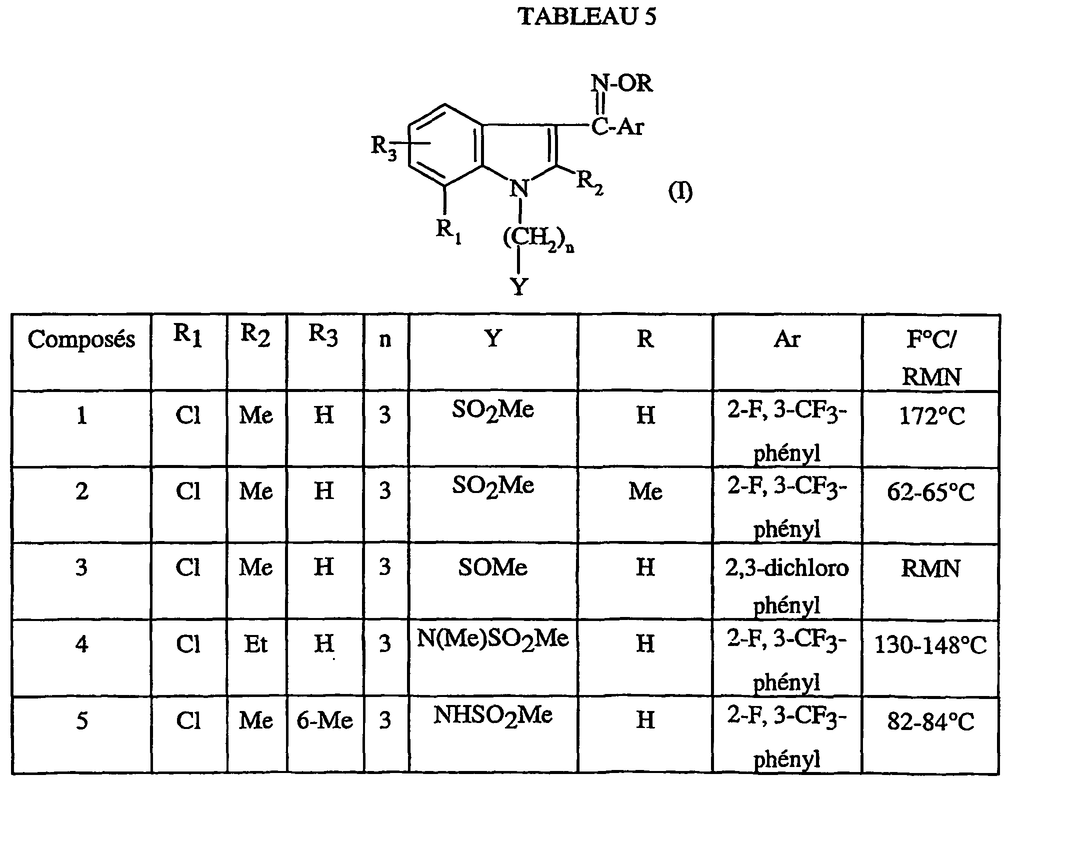

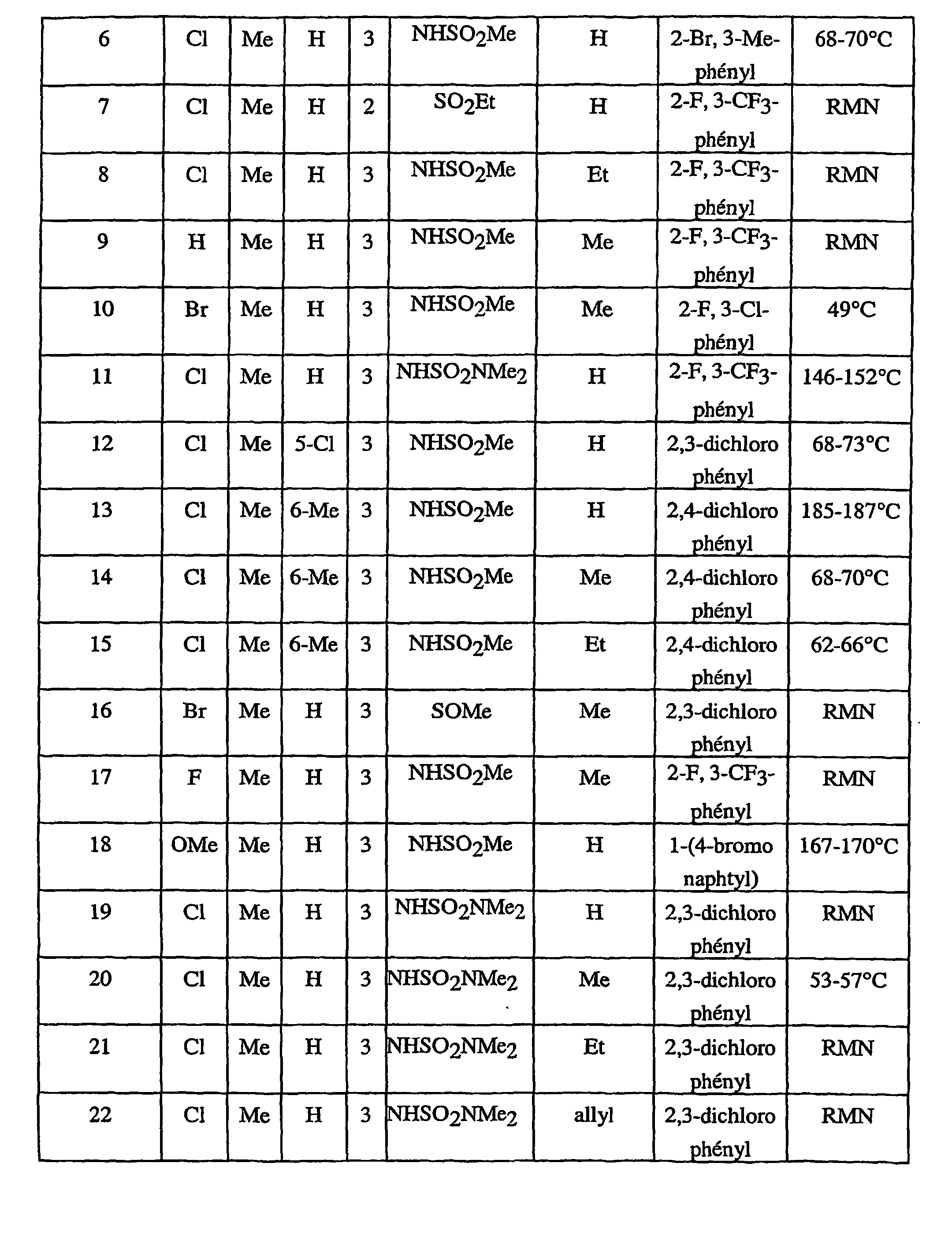

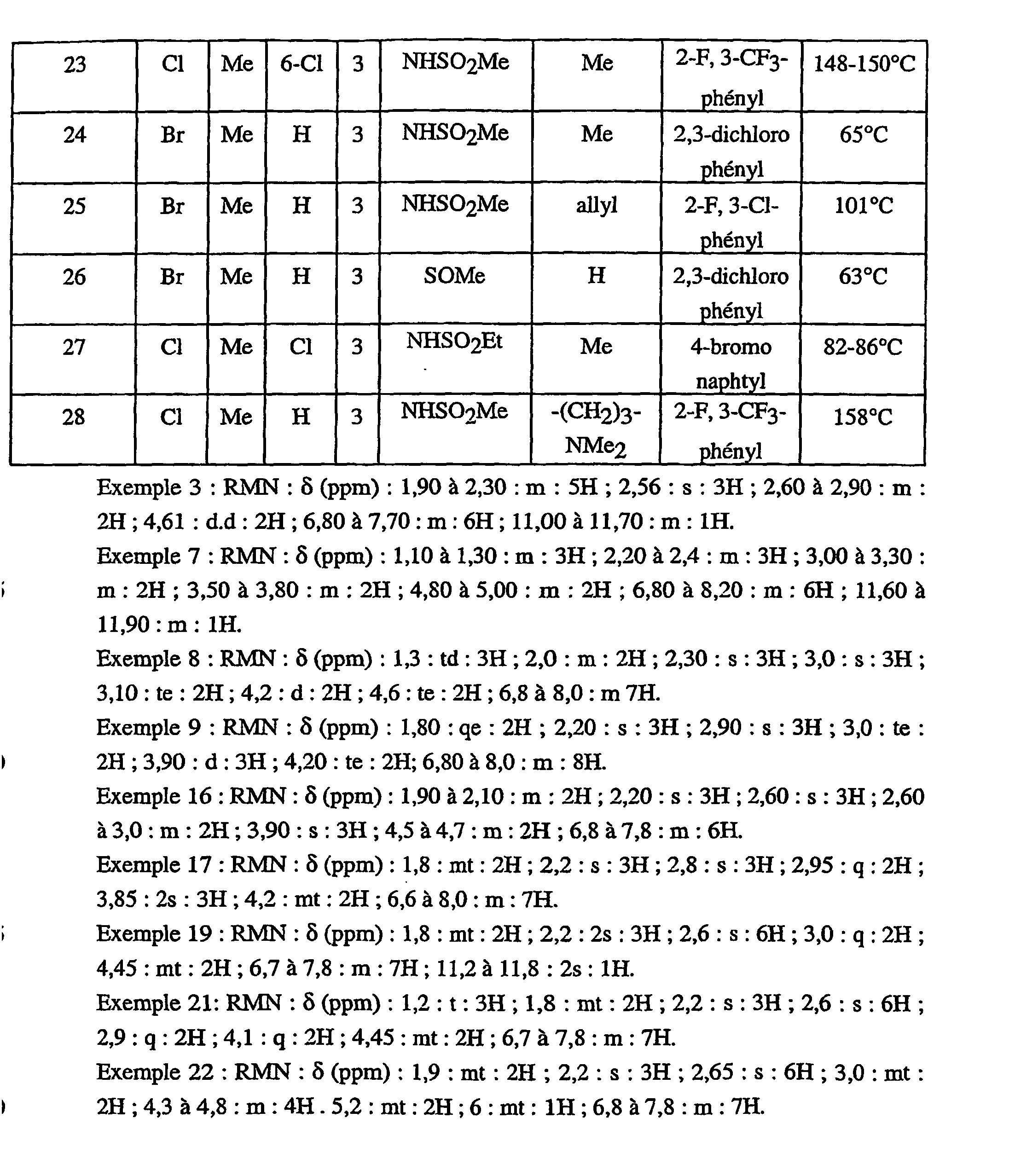

## Revendications

1. Un composé de formule : 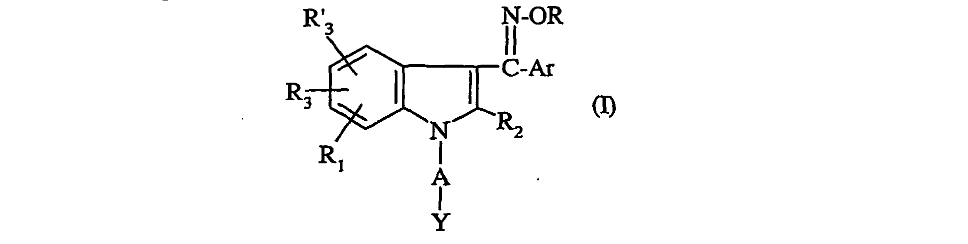 dans laquelle :
- Ar représente :
a) un phényle mono, di ou trisubstitué par un ou plusieurs groupes choisis parmi : un atome d'halogène, un groupe (C₁-C₄)alkyle, trifluorométhyle, amino, nitro, hydroxyle, (C₁-C₄)alcoxy, (C₁-C₄)alkylsulfanyle, (C₁-C₄)alkylsulfonyle ;
b) un naphtyle non substitué ou substitué une ou deux fois par un atome d'halogène, un groupe (C₁-C₄)alkyle ou trifluorométhyle ;
- A représente un radical alkylène en C₂-C₆ ;
- Y représente un groupe choisi parmi SR₄, SOR₄, SO₂R₄, SO₂NR₅R₆, N(R₇)SO₂R₄, OR₄, NR₇SO₂NR₅R₆ ;
- R représente l'hydrogène, un groupe (C₁-C₄)alkyle ou (C₂-C₄)alcényle ou un groupement (C₂-C₄)alkNR₈R₉ ;
- R₁, R₃ et R'₃ représentent chacun indépendamment l'un de l'autre l'hydrogène, un atome d'halogène, un groupe hydroxyle, (C₁-C₄)alkyle, trifluorométhyle, (C₁-C₄)alcoxy ;
- R₂ représente l'hydrogène ou un groupe (C₁-C₄)alkyle ;
- R₄ représente un groupe (C₁-C₄)alkyle ou un trifluorométhyle ;
- R₅ et R₆ représentent chacun indépendamment l'hydrogène ou un groupe (C₁-C₄)alkyle ;
- R₇ représente l'hydrogène ou un (C₁-C₄)alkyle ;
- R₈ et R₉ représentent chacun indépendamment l'un de l'autre l'hydrogène ou un groupe (C₁-C₄)alkyle ou R₈ et R₉ ensemble avec l'atome d'azote auquel ils sont liés constituent un radical hétérocyclique comportant de 4 à 7 chaînons et pouvant contenir un autre hétéroatome choisi parmi un atome d'azote, d'oxygène ou de soufre, non substitué ou substitué par un ou plusieurs groupes méthyle ou méthoxy ;
ainsi que ses sels éventuels et/ou ses solvats.

2. Un composé selon la revendication 1 de formule (I) dans laquelle :
- Ar représente :
a) un phényle mono, di ou trisubstitué par un ou plusieurs groupes choisis parmi : un atome d'halogène, un groupe (C₁-C₄)alkyle, trifluorométhyle, amino, nitro, (C₁-C₄)alcoxy, (C₁-C₄)alkylsulfanyle, (C₁-C₄)alkylsulfonyle ;
b) un naphtyle non substitué ou substitué une ou deux fois par un atome d'halogène, un groupe (C₁-C₄)alkyle ou trifluorométhyle ;
- A représente un groupe (CH₂)ₙ avec n représente 2, 3 ou 4 ;
- Y représente un groupe choisi parmi SR₄, SOR₄, SO₂R₄, SO₂NR₅R₆, N(R₇)SO₂R₄, OR₄ ;
- R représente l'hydrogène ou un groupe (C₁-C₄)alkyle ou (C₂-C₄)alcényle ou un groupement (C₂-C₄)alkNR₈R₉ ;
- R₁ est en position 7 du noyau indole et représente un atome d'halogène ou un groupe (C₁-C₄)alkyle, trifluorométhyle, (C₁-C₄)alcoxy ;
- R₂ représente l'hydrogène ou un groupe (C₁-C₄)alkyle ;
- R₃ représente l'hydrogène, un atome d'halogène ou un groupe (C₁-C₄)alkyle ;
- R'₃ est l'hydrogène ;
- R₄ représente un (C₁-C₄)alkyle ;
- R₅ et R₆ représentent chacun indépendamment l'hydrogène ou un groupe (C₁-C₄) alkyle ;
- R₇ représente l'hydrogène ou un (C₁-C₄)alkyle ;
- R₈ et R₉ représentent un (C₁-C₄)alkyle ou ensemble avec l'atome d'azote auquel ils sont liés constituent un radical hétérocyclique choisi parmi : azétidinyle, pyrrolidinyle, pipéridinyle, perhydroazépinyle, pipérazinyle, 4-méthylpipérazin-1-yle, morpholino, thiomorpholino ;
ainsi que ses sels éventuels et/ou ses solvats.

3. Un composé selon la revendication 1 de formule (I) dans laquelle :
- Ar représente un phényle mono ou disubstitué par un atome d'halogène, un groupe méthyle, trifluorométhyle, méthoxy, méthylsulfanyle, méthylsulfonyle ;
- A représente un groupe (CH₂)ₙ avec n représente 2, 3 ou 4 ;
- Y représente un groupe SO₂R₄ ou NHSO₂R₄ ;
- R₁ représente un groupe méthyle, un atome de chlore ou de brome, en position 7 du noyau indole ;
- R₂ représente un groupe méthyle ;
- R₃ est l'hydrogène ou R₃ est en position 6 du noyau indole et représente soit un atome de chlore, soit un groupe méthyle ;
- R'₃ est l'hydrogène ;
- R₄ représente un groupe méthyle ou éthyle ;
- R représente l'hydrogène, un groupe méthyle ou éthyle ou un groupement (CH₂)₃N(CH₃)₂ ;
ainsi que ses sels éventuels et/ou ses solvats.

4. Procédé de préparation d'un composé de formule (I) selon la revendication 1, de ses sels éventuels et/ou ses solvats, **caractérisé en ce que** l'on traite un dérivé d' aroylindole de formule : 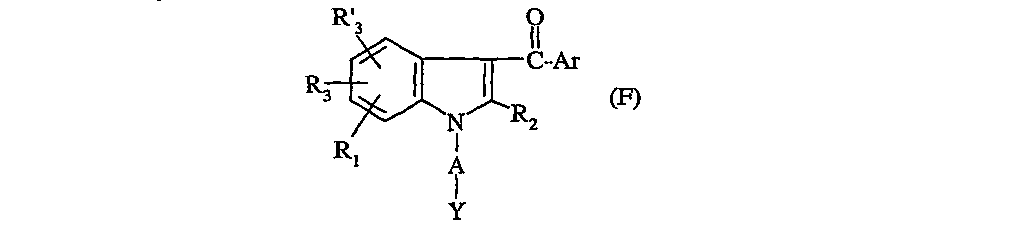 dans laquelle R₁, R₂, R₃, R'₃, A, Y et Ar sont tels que définis dans la revendication 1 par un dérivé d'hydroxylamine de formule H₂NOR dans laquelle R est tel que défini dans la revendication 1.

5. Composition pharmaceutique contenant en tant que principe actif un composé selon l'une quelconque des revendications 1 à 3.

6. Composition pharmaceutique selon la revendication 5, contenant de 0,1 à 1000 mg de principe actif, sous forme d'unité de dosage dans laquelle le principe actif est mélangé à au moins un excipient pharmaceutique.

7. Médicament **caractérisé en ce qu'**il est constitué d'un composé selon l'une quelconque des revendications 1 à 3.

8. Utilisation d'un composé selon l'une quelconque des revendications 1 à 3 pour la préparation de médicaments destinés à combattre toute pathologie dans laquelle les récepteurs aux cannabinoïdes CB₂ sont impliqués.

9. Utilisation d'un composé selon l'une quelconque des revendications 1 à 3 pour la préparation de médicaments destinés à combattre les maladies autoimmunes, les maladies allergiques, les maladies infectieuses, les maladies neurodégénératives, les maladies cardiovasculaires, les cancers, les maladies gastro-intestinales, l'obésité, le diabète.

## Patentansprüche

1. Verbindung der Formel: 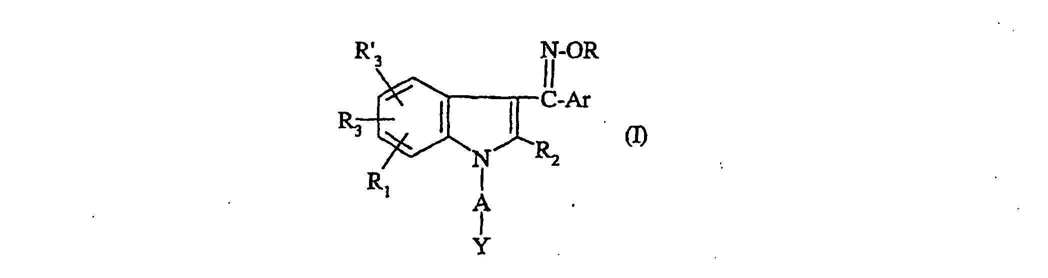 in der:
- Ar:
(a) ein Phenyl, das durch eine oder mehrere Gruppen, ausgewählt unter: einem Halogenatom, einer (C₁₋₄)-Alkyl-, Trifluormethyl-, Amino-, Nitro-, Hydroxyl-, (C₁₋₄)Alkoxy-, (C₁₋₄)Alkylsulfanyl-, (C₁₋₄)Alkylsulfonyl-Gruppe, mono-, di- oder trisubstituiert ist;
(b) ein Naphthyl, das unsubstituiert oder ein oder zweimal mit einem Halogenatom, einer (C₁₋₄)Alkyl- oder Trifluormethylgruppe substituiert ist, darstellt;
- A einen (C₂₋₆)Alkylenrest darstellt;
- Y eine Gruppe, ausgewählt unter SR₄, SOR₄, SO₂R₄, SO₂NR₅R₆, N(R₇)SO₂R₄, OR₄, NR₇SO₂NR₅R₆, darstellt;
- R Wasserstoff, eine (C₁₋₄)Alkyl- oder (C₂₋₄)-Alkenylgruppe oder eine (C₂₋₄)AlkNR₈R₉-Gruppierung darstellt;
- R₁, R₃ und R'₃ jeweils unabhängig voneinander Wasserstoff, ein Halogenatom, eine Hydroxylgruppe, (C₁₋₄)Alkyl, Trifluormethyl, (C₁₋₄)Alkoxy darstellen;
- R₂ Wasserstoff oder eine (C₁₋₄)Alkylgruppe darstellt;
- R₄ eine (C₁₋₄)Alkyl- oder eine Trifluormethylgruppe darstellt;
- R₅ und R₆ unabhängig voneinander Wasserstoff oder eine (C₁₋₄)Alkylgruppe darstellen;
- R₇ Wasserstoff oder ein (C₁₋₄)Alkyl darstellt;
- R₈ und R₉ jeweils unabhängig voneinander Wasserstoff oder eine (C₁₋₄)Alkylgruppe darstellen oder R₈ und R₉ zusammen mit dem Stickstoffatom, an das sie gebunden sind, einen heterocyclischen Rest bilden, der 4 bis 7 Glieder trägt und ein anderes Heteroatom, ausgewählt aus einem Stickstoffatom, Sauerstoffatom oder Schwefelatom, enthalten kann, nicht substituiert oder durch eine oder mehrere Methyl- oder Methoxy-Gruppen substituiert sein kann;
sowie ihre möglichen Salze und/oder ihre Solvate.

2. Verbindung nach Anspruch 1 der Formel (I), in der:
- Ar:
(a) ein Phenyl, das durch eine oder mehrere Gruppen, ausgewählt unter: einem Halogenatom, einer (C₁₋₄)-Alkyl-, Trifluormethyl-, Amino-, Nitro-, (C₁₋₄)-Alkoxy-, (C₁₋₄)Alkylsulfanyl-, (C₁₋₄)-Alkylsulfonyl-Gruppe, mono-, di- oder tri-substituiert ist;
(b) ein Naphthyl, das unsubstituiert oder ein oder zweimal mit einem Halogenatom, einer (C₁₋₄)Alkyl- oder Trifluormethylgruppe substituiert ist, darstellt;
- A eine (CH₂)ₙ-Gruppe, worin n 2, 3 oder 4 bedeutet, darstellt:
- Y eine Gruppe, ausgewählt unter SR₄, SOR₄, SO₂R₄, SO₂NR₅R₆, N(R₇)SO₂R₄, OR₄, darstellt;
- R Wasserstoff oder eine (C₁₋₄) -Alkyl- oder (C₂₋₄)-Alkenylgruppe oder eine (C₂₋₄)-AlkNR₈R₉-Gruppierung darstellt;
- R₁ in Position 7 des Indolrings ist und ein Halogenatom oder eine (C₁₋₄)Alkyl-, Trifluormethyl-, (C₁₋₄)Alkoxy-Gruppe darstellt;
- R₂ Wasserstoff oder eine (C₁₋₄)Alkylgruppe darstellt;
- R₃ Wasserstoff, ein Halogenatom oder eine (C₁₋₄)-Alkylgruppe darstellt;
- R'₃ Wasserstoff ist;
- R₄ ein (C₁₋₄)Alkyl darstellt;
- R₅ und R₆ unabhängig voneinander jeweils Wasserstoff oder eine (C₁₋₄)Alkylgruppe darstellen;
- R₇ Wasserstoff oder ein (C₁₋₄)Alkyl darstellt;
- R₈ und R₉ ein (C₁₋₄)Alkyl darstellen oder zusammen mit dem Stickstoffatom, an das sie gebunden sind, einen heterocyclischen Rest darstellen, der aus Azetidinyl, Pyrrolidinyl, Piperidinyl, Perhydroazepinyl, Piperazinyl, 4-Methylpiperazin-1-yl, Morpholino, Thiomorpholino ausgewählt ist;
sowie ihre möglichen Salze und/oder ihre Solvate.

3. Verbindung nach Anspruch 1 der Formel (I), in der:
- Ar ein Phenyl darstellt, das durch ein Halogenatom, eine Methyl-, Trifluormethyl-, Methoxy-, Methylsulfanyl-, Methylsulfonyl-Gruppe mono- oder disubstituiert ist;
- A eine (CH₂)ₙ-Gruppe, worin n für 2, 3 oder 4 steht, darstellt;
- Y eine SO₂R₄- oder NHSO₂R₄-Gruppe darstellt;
- R₁ eine Methylgruppe, ein Chlor- oder Bromatom in Position 7 des Indolkerns darstellt;
- R₂ eine Methylgruppe darstellt;
- R₃ Wasserstoff ist oder R₃ in Position 6 des Indolkerns ist und entweder ein Chloratom oder eine Methylgruppe darstellt;
- R'₃ Wasserstoff ist;
- R₄ eine Methyl- oder Ethylgruppe darstellt;
- R Wasserstoff, eine Methyl- oder Ethylgruppe oder eine -(CH₂)₃N(CH₃)₂-Gruppierung darstellt;
sowie ihre möglichen Salze und/order ihre Solvate.

4. Verfahren zur Herstellung einer Verbindung der Formel (I) nach Anspruch 1, ihrer möglichen Salze und/oder ihrer Solvate, **dadurch gekennzeichnet, dass** man ein Aroylindolderivat der Formel: 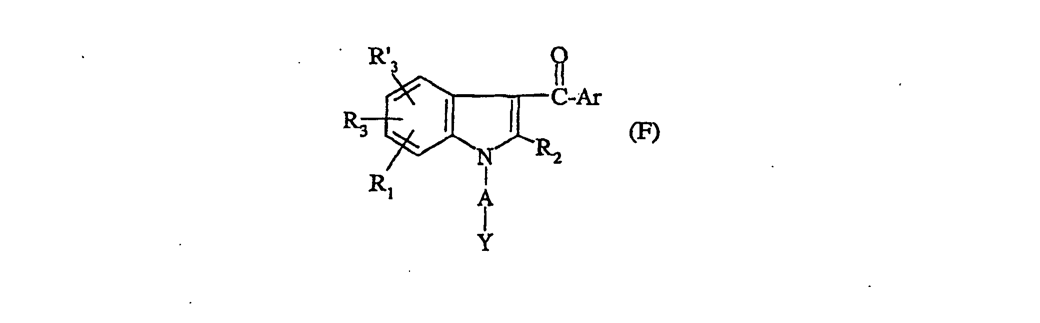 in der R₁, R₂, R₃, R'₃, A, Y und Ar wie in Anspruch 1 definiert sind, mit einem Hydroxylaminderivat der Formel H₂NOR, worin R wie in Anspruch 1 definiert ist, behandelt.

5. Pharmazeutische Zusammensetzung, die als Wirkstoff eine Verbindung nach einem der Ansprüche 1 bis 3 enthält.

6. Pharmazeutische Zusammensetzung nach Anspruch 5, die 0,1 bis 1.000 mg Wirkstoff in Dosierungseinheitsform enthält, in der der Wirkstoff mit wenigstens einem pharmazeutischen Exzipiens vermischt ist.

7. Medikament, **dadurch gekennzeichnet, dass** es aus einer Verbindung nach einem der Ansprüche 1 bis 3 besteht.

8. Verwendung einer Verbindung nach einem der Ansprüche 1 bis 3 zur Herstellung von Medikamenten, die dazu bestimmt sind, jede Pathologie zu bekämpfen, in der die Rezeptoren für Cannabinoide CB₂ involviert sind.

9. Verwendung einer Verbindung nach einem der Ansprüche 1 bis 3 zur Herstellung von Medikamenten, die dazu bestimmt sind, Autoimmunkrankheiten, allergische Krankheiten, Infektionskrankheiten, neurodegenerative Krankheiten, kardiovaskuläre Krankheiten, Krebs, gastrointestinale Krankheiten, Fettsucht, Diabetes zu bekämpfen.

## Claims

1. Compound of formula: 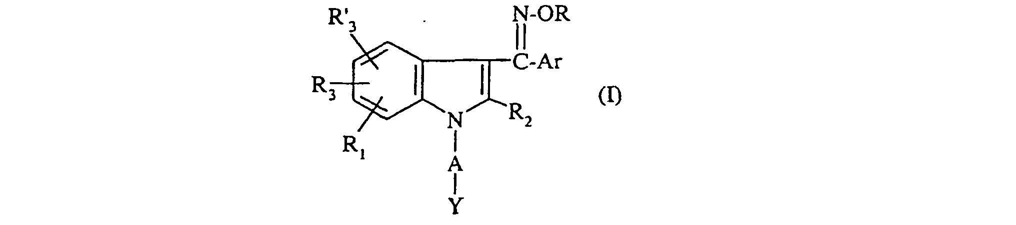 in which:
- Ar represents:
a) a phenyl which is mono-, di- or trisubstituted by one or more groups selected from the following: a halogen atom, a (C₁-C₄)alkyl, trifluoromethyl, amino, nitro, hydroxyl, (C₁-C₄)alkoxy, (C₁-C₄)alkylsulphanyl, or (C₁-C₄)alkylsulphonyl group;
b) a naphthyl which is unsubstituted or substituted once or twice by a halogen atom, a (C₁-C₄)alkyl group or a trifluoromethyl;
- A represents a C₂-C₆ alkylene radical;
- Y represents a group selected from SR₄, SOR₄, SO₂R₄, SO₂NR₅R₆, N(R₇)SO₂R₄, OR₄ and NR₇SO₂NR₅R₆;
- R represents hydrogen, a (C₁-C₄)alkyl or (C₂-C₄)alkenyl group or a group (C₂-C₄)alk-NR₈R₉;
- R₁, R₃ and R'₃ represent each independently of one another hydrogen, a halogen atom or a hydroxyl, (C₁-C₄)alkyl, trifluoromethyl or (C₁-C₄)alkoxy group;
- R₂ represents hydrogen or a (C₁-C₄)alkyl group;
- R₄ represents a (C₁-C₄)alkyl group or a trifluoromethyl;
- R₅ and R₆ represent each independently hydrogen or a (C₁-C₄)alkyl group;
- R₇ represents hydrogen or a (C₁-C₄)alkyl group;
- R₈ and R₉ represent each independently of one another hydrogen or a (C₁-C₄)alkyl group, or R₈ and R₉ together with the nitrogen atom to which they are connected constitute a heterocyclic radical containing from 4 to 7 ring members and being able to contain another heteroatom selected from a nitrogen, oxygen or sulphur atom, unsubstituted or substituted by one or more methyl or methoxy groups;
and its salts, where appropriate, and/or its solvates.

2. Compound according to Claim 1 of formula (I) in which:
- Ar represents:
a) a phenyl which is mono-, di- or trisubstituted by one or more groups selected from the following: a halogen atom, a (C₁-C₄)alkyl, trifluoromethyl, amino, nitro, (C₁-C₄)alkoxy, (C₁-C₄)alkylsulphanyl, or (C₁-C₄)alkylsulphonyl group;
b) a naphthyl which is unsubstituted or substituted once or twice by a halogen atom, a (C₁-C₄)alkyl group or a trifluoromethyl;
- A represents a group (CH₂)ₙ where n represents 2, 3 or 4;
- Y represents a group selected from SR₄, SOR₄, SO₂R₄, SO₂NR₅R₆, N(R₇)SO₂R₄ and OR₄;
- R represents hydrogen, a (C₁-C₄)alkyl or (C₂-C₄)alkenyl group or a group (C₂-C₄)alk-NR₈R₉;
- R₁ is in position 7 of the indole ring system and represents a halogen atom or a (C₁-C₄)alkyl, trifluoromethyl or (C₁-C₄)alkoxy group;
- R₂ represents hydrogen or a (C₁-C₄)alkyl group;
- R₃ represents hydrogen, a halogen atom or a (C₁-C₄)alkyl group;
- R'₃ is hydrogen;
- R₄ represents a (C₁-C₄)alkyl;
- R₅ and R₆ represent each independently hydrogen or a (C₁-C₄)alkyl group;
- R₇ represents hydrogen or a (C₁-C₄)alkyl;
- R₈ and R₉ represent a (C₁-C₄)alkyl or, together with the nitrogen atom to which are connected, constitute a heterocyclic radical selected from: azetidinyl, pyrrolidinyl, piperidinyl, perhydroazepinyl, piperazinyl, 4-methylpiperazin-1-yl, morpholino and thiomorpholino;
and its salts, where appropriate, and and/or its solvates.

3. Compound according to Claim 1 of formula (I) in which:
- Ar represents a phenyl which is mono- or disubstituted by a halogen atom or a methyl, trifluoromethyl, methoxy, methylsulphanyl or methylsulphonyl group;
- A represents a group (CH₂)ₙ where n represents 2, 3 or 4;
- Y represents a group SO₂R₄ or NHSO₂R₄ ;
- R₁ represents a methyl group or a chlorine or bromine atom in position 7 of the indole ring system;
- R₂ represents a methyl group;
- R₃ is hydrogen or R₃ is in position 6 of the indole ring system and represents either a chlorine atom or a methyl group;
- R'₃ is hydrogen;
- R₄ represents a methyl or ethyl group;
- R represents hydrogen, a methyl or ethyl group or a
- (CH₂)₃N(CH₃)₂ moiety;
and its salts, where appropriate, and/or its solvates.

4. Process for preparing a compound of formula (I) according to Claim 1, its salts, where appropriate, and/or its solvates, **characterized in that** an aroylindole derivative of formula: 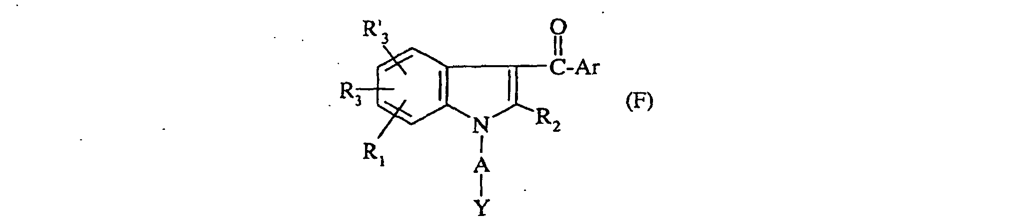 in which R₁, R₂, R₃, R'₃, A, Y and Ar are as defined in Claim 1 is treated with a hydroxylamine derivative of formula H₂NOR in which R is as defined in Claim 1.

5. Pharmaceutical composition comprising as active principle a compound according to any one of Claims 1 to 3.

6. Pharmaceutical composition according to Claim 5, containing from 0.1 to 1000 mg of active principle, in a unit dosage form in which the active principle is mixed with at least one pharmaceutical excipient.

7. Medicinal product **characterized in that** it is composed of a compound according to any one of Claims 1 to 3.

8. Use of a compound according to any one of Claims 1 to 3 for preparing medicinal products intended for combating any pathology in which CB₂ cannabinoid receptors are involved.

9. Use of a compound according to any one of Claims 1 to 3 for preparing medicinal products intended for combating autoimmune diseases, allergic diseases, infectious diseases, neurodegenerative diseases, cardiovascular diseases, cancers, gastrointestinal diseases, obesity and diabetes.
